# EUROPEAN PATENT SPECIFICATION

(11) **EP 4 366 787 B1**
(45) Date of publication and mention of the grant of the patent: **11.06.2025**
(21) Application number: 22735000.6
(22) Date of filing: 30.06.2022
(51) Int. Cl.: A61L 2/08, A01N 63/20, A01N 63/40, A61L 2/10, A61L 2/24, A61L 9/013, A61L 9/14, F21V 33/00, A61L 2/00, A01P 1/00

(54) **INDOOR MICROBIOME MANAGEMENT**
VERWALTUNG EINES MIKROBIOMS IN INNENRÄUMEN
GESTION DU MICROBIOME INTÉRIEUR

(30) Priority: 06.07.2021 US 202163218544 P; 20.07.2021 EP 21186758; 18.10.2021 US 202163256832 P
(43) Date of publication of application: 15.05.2024
(73) Proprietor: Signify Holding B.V., 5656 AE Eindhoven (NL)
(72) Inventor: DEIXLER, Peter, 5656 AE Eindhoven (NL)
(74) Representative: Verweij, Petronella Daniëlle
(86) International application number: PCT/EP2022/068011
(87) International publication number: WO 2023/280665

(56) References cited:
- EP-A1- 4 146 989
- EP-A1- 4 369 919
- WO-A1-2019/025808
- WO-A1-2021/250671
- CN-A- 117 615 796
- LI SHUAI ET AL: "Understanding building-occupant-microbiome interactions toward healthy built environments: A review", FRONTIERS OF ENVIRONMENTAL SCIENCE, HIGHER EDUCATION PRESS, BEIJING, vol. 15, no. 4, 22 October 2020 (2020-10-22), XP037285000, ISSN: 2095-2201, [retrieved on 20201022], DOI: 10.1007/S11783-020-1357-3
- HORVE PATRICK F ET AL: "Building upon current knowledge and techniques of indoor microbiology to construct the next era of theory into microorganisms, health, and the built environment", JOURNAL OF EXPOSURE SCIENCE AND ENVIRONMENTAL EPIDEMIOLOGY, NATURE PUBLISHING GROUP US, NEW YORK, vol. 30, no. 2, 15 July 2019 (2019-07-15), pages 219 - 235, XP037065343, ISSN: 1559-0631, [retrieved on 20190715], DOI: 10.1038/S41370-019-0157-Y

## Description

### FIELD OF THE INVENTION

The invention relates to a lighting system for indoor microbiome management. The invention further relates to a lighting device comprising the lighting system. The invention further relates to a method for indoor microbiome management.

### BACKGROUND OF THE INVENTION

Methods for indoor microbiome management are known in the art. For instance, US2008107699A1 describes a method and composition for application of probiotic microorganisms to a surface to prevent contamination by pathogenic microorganisms. The probiotic microorganisms may be bacteria, yeast or mold, such as bacteria of the *Lactobacillus* genus. Application of probiotic microorganisms may be to surfaces such as of the hands, or to other surfaces such as tables, equipment, clothing and the like. WO2019/025808A1 discloses a bioreactor. WO2021250671A1 and EP4146989A1 (Art. 54(3) EPC) disclose an air treatment system using an aerosolized probiotic solution.

### SUMMARY OF THE INVENTION

In large parts of the world, humans may spend about 90% of their time indoors. For years scientists have sounded the alarm that our disconnect from the outdoors may be linked to a host of chronic health problems, including allergies, asthma, depression, irritable bowel syndrome, and obesity. In particular, it may appear that buildings, even those buildings designed to be as germ-free as possible, may actually be vectors for disease (i.e. due to the extremely clean surfaces allowing any harmful germs to grow unusually quickly), not the least Covid-19 and hospital-acquired infections. Like our bodies, the buildings we inhabit are also teeming with microbes. Historically, research may have primarily focused on problematic/undesirable microbes, such as black mold and legionella. However, the indoor environment may comprise rich ecologies of fast-evolving indoor microbe populations. These microbe populations may, however, have little overlap with outdoor microbe populations, including salutary species that humans co-evolved with over millions of years. Hence, the prior art may describe the disinfection of indoor environments (and objects) to reduce exposure to pathogens.

The prior art may further describe healthy building design guidelines that teach to use air disinfection only during the day hours, but when people are gone to actively recharge the building with "good" microbes from the outside air. Some class-A commercial buildings may deploy ventilation systems that are configured to pump microbially diverse outdoor air into office spaces. However, many office buildings may be located in (large) cities, rather than in in environments with easy access to clean and/or natural outdoor air. Hence, for instance, the 31-story WeBank tower in Shenzhen is planned upon completion in 2022 to draw air through trees planted on balconies before the air is funneled to the inside of the building. This approach may, however, be unpractical for the mass-market of common buildings due to the high cost of maintaining trees on the building facade. The prior art may further comprise probiotic cleaners or air enhancers that actively apply soil/ocean microbes into a room. These microbes may, however, struggle to settle into indoor environments already inhabited by other microbes, which may potentially be more attuned to the indoor environment.

Further, in the last decades, the need for disinfection systems may have been challenged, as disinfection also kills off the wanted microbiome in the building, which may be harder to restore, similar to the use of antibiotics when treating an infection. However, in the modem world with high population densities and frequent travel, and also in view of deadly disease outbreaks such as of COVID-19, providing safe indoor environments may be crucial, for community centers, hospitals, offices, and even homes; active disinfection of indoor environments may thus (continue to) be important in preventing disease outbreaks.

The prior art may further describe recharging an indoor biome with microbes from outside by opening up the buildings at night when air conditioning is not required. In a typical modern city, the outdoor air may be heavily polluted and the air may thus need to be heavily filtered before being pumped indoors. However, many beneficial outdoor microbes are removed by the filtering or may not survive getting pumped through, for example, a heating, ventilation and air conditioning (HVAC; Heating, ventilation, and air conditioning) duct. Even in less polluted cities, many modem office buildings may not have operable windows to let fresh air in. Hence, for many office buildings, direct use of outdoor air may realistically not be a suitable option for consistently introducing outdoor microbes.

Another issue in the built environment, especially for offices and public spaces, is that biofilms may develop on surfaces of said built environment. A biofilm is a very thin and slimy coating of bacteria and polymers that clings to surfaces. For example, often noticed as a yellowish tarnish or stain on ceiling plates, walls, or wallpaper. It is known that many microorganisms can develop biofilms attached to common building surface materials such as wood, linoleum, ceramic, and stainless steel. Surface-associated microorganisms capable of forming biofilms include many bacteria, fungi and protists. Hence, a clear need is present to automatically and frequently remove a biofilm on building surfaces.

Hence, it is an aspect of the invention to provide an alternative system for indoor microbiome management, which preferably further at least partly obviates one or more of above-described drawbacks. The present invention may have as object to overcome or ameliorate at least one of the disadvantages of the prior art, or to provide a useful alternative.

The present invention is set out in the appended independent and dependent claims.

Hence, in a first aspect, the invention provides a lighting system, for indoor microbiome management. The system comprises a light generating device and a microbe dispenser device. The microbe dispenser device is configured to provide in a microbic application mode (of the system) a (microbe) emission of first microbes, especially wherein the microbe dispenser device has a microbe emission region. The light generating device is configured to provide in a microbic lighting mode (of the system) a first beam of first device radiation. In particular, a spectral power distribution of the first device radiation is selected for promoting persistence, especially growth, of the first microbes relative to second microbes, other than the first microbes. The microbe emission region and the first beam at least partly spatially overlap.

The system of the invention may thus both provide (beneficial) first microbes and provide first device radiation suitable for promoting the persistence of the first microbes relative to second microbes. In particular, the first device radiation may be beneficial for the growth of the first microbes, and/or may be detrimental to the growth of the second microbes. Thereby, the first device radiation may provide the first microbes a competitive advantage over the second microbes, allowing the first microbes to settle and persist in an indoor environment.

In particular, the system (and the method; see below) of the invention may actively enhance microbial diversity throughout an indoor environment, including by deliberate introduction of wanted microbial species, such as those commonly found in nature on plants and leaves. These microbes may further mitigate the side effects of (overzealous) disinfection of the room, for instance with light-based disinfection or ionizers, and may provide competition for harmful microbes arriving later.

Hence, the invention may relate to both dispensing (or "applying") microbes and further promoting the wanted microbiome in the room with the help of tailored lighting recipes. **In** embodiments (see below), the microbe dispensers may be actuated depending on a current context of an indoor environment as well as depending on the past/current/predicted operation status of a disinfection system and the room's (expected) occupancy status or (expected) activity in the room.

In particular, the zest of the invention may be to selectively promote a first microbe (which, for instance may be known to be relatively robust against a selected light wavelength) vs a second microbe which is more susceptible to the selected wavelength, such as 222 nm. The first microbe may also be damaged or deactivated by the selected wavelength, but to a lesser degree than the second microbe, such as due to a lower absorption of radiation at the selected wavelength, or such as due to better or more active repair mechanisms. Hence, the selected wavelength may shift the balance between the first and second microbe, helping the first microbe to dominate on the surface via a competitive exclusion mechanism.

The invention provides a lighting system for indoor microbiome management, wherein the system comprises a light generating device and a microbe dispenser device; wherein: the microbe dispenser device is configured to provide in a microbic application mode an emission of first microbes, wherein the microbe dispenser device has a microbe emission region; the light generating device is configured to provide in a microbic lighting mode a first beam of first device radiation (or "first radiation"); a spectral power distribution of the first device radiation is selected for promoting persistence of the first microbes relative to second microbes, other than the first microbes; and the microbe emission region and the first beam at least partly spatially overlap.

Hence, the invention provides a lighting system for indoor microbiome management.

The term "indoor microbiome" may herein refer to the collection of microbes in a built environment, especially in an indoor environment. In general, the indoor microbiome may comprise a plurality of different species, including bacteria, archaea, and fungi. In particular, an indoor environment may host a plurality of different indoor microbiomes together forming a total indoor microbiome. For instance, the microbiome on a table surface may differ from that of a floor surface, and both may differ from that on a windowsill.

**In** embodiments, the indoor microbiome may especially be a surface indoor microbiome, such as the microbiome of one or more of a table, a floor, a wall, or a ceiling.

**In** further embodiments, the indoor microbiome may be a (total) room indoor microbiome, i.e., the indoor microbiome may comprise all microbes in a room.

The system comprises a microbe dispenser device. The microbe dispenser device is especially configured to provide an emission of first microbes, i.e., the microbe dispenser device may be configured to continuously, periodically, or intermittently provide first microbes to an indoor environment. **In** embodiments, the microbe dispenser device may especially be configured to provide in the microbic application mode a spray of the first microbes. In further embodiments, the microbe dispenser device may especially be configured to provide in the microbic application mode a (dry) powder of the first microbes, i.e., to provide the first microbes in a powder form.

The first microbes (or: "first micro-organisms") may especially comprise beneficial microbes, including microbes with (direct) health benefits, but also microbes that may compete with pathogenic (or otherwise undesirable) microbes and may thereby (indirectly) provide health benefits.

In embodiments, the first microbes may be selected from the group comprising the genera *Akkermansia, Bifidobacterium, Lachnospira, Alistipes, Bacteroides, Coprococcus, Viellonella, Faecalibacterium, Roseburia, Dialister, Sutterella, Methanobrevibacter, Lactobacillus, Parabacteroides, Prevotella, Agathobacter, Eubacterium, Ruminococcus, Nitrosomonas, Nitrosospira, Nitrosopumilus, Cenarchaeum, Nitrosoarchaeum, Nitrosocaldus, Caldiarchaeum, Acinetobacter, Alcaligenes, Arthrobacter, Azospirillum, Azotobacter, Bacillus, Beijerinckia, Enterobacter, Erwinia, Flavobacterium, Rhizobium, Serratia* and *Deinococcus.* The first microbes may especially comprise a plurality of different species, such as different genera. The terms "first microbe", or "first microbes", and similar terms, may refer to one or more different types of microbes. In further embodiments, the first microbes may comprise (species belonging to) two or more of the genera*Akkermansia, Bifidobacterium, Lachnospira, Alistipes, Bacteroides, Coprococcus, Viellonella, Faecalibacterium, Roseburia, Dialister, Sutterella, Methanobrevibacter, Lactobacillus, Parabacteroides, Prevotella, Agathobacter, Eubacterium, Ruminococcus, Nitrosomonas, Nitrosospira, Nitrosopumilus, Cenarchaeum, Nitrosoarchaeum, Nitrosocaldus, Caldiarchaeum, Acinetobacter, Alcaligenes, Arthrobacter, Azospirillum, Azotobacter, Bacillus, Beijerinckia, Enterobacter, Erwinia, Flavobacterium, Rhizobium, Serratia* and *Deinococcus.* Hence, the first microbes may comprise a microbial community of two or more species. In further embodiments, the first microbes may be selected from the group comprising the species RF39, *Akkermansia mucinophila, Bifidobacterium longum, Roseburia inulinivorans* and *Faecalibacterium prausnitzii.* In specific embodiments, the first microbes may comprise (at least) *Bacillus.*

In particular, the microbe dispenser device is configured to have a microbe emission region (or range), i.e., the microbe dispenser device may (be configured to) provide the first microbes to a microbe emission region. In embodiments, the microbe emission region may be a 2D region, such as a surface. In further embodiments, the microbe emission region may be a 3D region, such as an (indoor) space, especially a room.

Especially, in embodiments the microbe dispenser device is configured to provide in a microbic application mode an emission of first microbes and essentially no second microbes, i.e., the emission is (essentially) devoid of second microbes.

In embodiment, the system may have a control system configured to control one or more elements of the system. The term "controlling" and similar terms herein may especially refer at least to determining the behavior or supervising the running of an element. Hence, herein "controlling" and similar terms may e.g. refer to imposing behavior to the element (determining the behavior or supervising the running (or "activity") of an element), etc., such as e.g. measuring, displaying, actuating, opening, shifting, changing temperature, etc.. Beyond that, the term "controlling" and similar terms may additionally include monitoring. Hence, the term "controlling" and similar terms may include imposing behavior on an element and also imposing behavior on an element and monitoring the element. The controlling of the element can be done with a control system. The control system and the element may thus at least temporarily, or permanently, functionally be coupled. The element may comprise the control system. In embodiments, the control system and the element may not be physically coupled. Control can be done via wired and/or wireless control. The term "control system" may also refer to a plurality of different control systems, which especially are functionally coupled, and of which e.g. one master control system may be a control system and one or more others may be slave control systems.

In embodiments, the system, especially the control system, may have a microbic application mode. In the microbic application mode, the microbe dispenser device may (be configured to) provide a (microbe) emission of the first microbes. In particular, in the microbic application mode, the microbe dispenser device may (be configured to) provide the emission to the microbe emission region. Hence, during the microbic emission mode, the microbe emission region may be populated with the first microbes.

As partly mentioned above, one problem may be the formation of biofilms, due to undesired growth of surface-associated microorganisms in time. This may however be addressed by other probiotic cultures, which may for example successfully remove (in other words "eat up") the biofilms. Since such biofilms may be associated with building-smell, dust-mite allergies as well as the occurrence of infectious diseases (due to pathogens ability to hide within the biofilm from the regular cleaning disinfectants such as citric acid or ethanol), the use of probiotic microbe cultures may reduce and/or eliminate these problems associated with biofilms.

Hence, in an embodiment of the invention, said microbe emission region of the microbe dispenser device may be arranged to at least partly comprise a first surface. Alternatively phrased, said microbe emission region of the microbe dispenser device may be arranged to at least partly covers, or is directed to, a first surface. Said first surface may comprise a biofilm. Biofilm may be phrased as biofilm layer throughout the application. Hence, the microbe emission region may comprise a region comprising a biofilm. Said biofilm may comprise said second microbes. Said first microbes may be configured to dissolve, compete with and/or clean said second microbes. The applied (e.g. non-pathogenic) first microbes may fully replaces the resident biofilm-growing second microbes and associated pathogens.

Thereby, the first microbes may comprise for example Bacillus, which produce enzymes which are able to cut down biofilms covering building surfaces. For instance, probiotic microbes, such as e.g. the first microbes, can cut down on organic pollutions such as fat- or sweat-deposits on the building surfaces such as meeting tables or false ceilings. These biofilms may be associated with certain undesired odors (e.g. "sweat smell' in rooms). Upon the probiotic first microbes successfully removing the biofilm, the undesired smell is eliminated from the space, e.g. a building.

Even further, dust mite allergy within humans is known to be widespread. Dust mite allergy is an allergic reaction to the droppings of the dust mites. The dust mite droppings act as an indoor aero-allergen, which on inhalation triggers an allergic reaction in some humans. Biofilms may also be indirectly involved in dust mite allergy as dead dust mites, skin cells, and pet dander adhere to the biofilm and act as a food source for dust mites (and other bacteria) to feed off of and thrive from.

As mentioned, probiotic first microbes dispensed to building surfaces can break down the allergens produced by the dust mites, and may even disintegrate the biofilm which acts as the food source for bacteria and dust mites. After the probiotic microbes have successfully eliminated said food source, the dust mites can no longer survive. The application of a probiotic layer with the microbe dispenser device according to the invention (i.e. provided to the microbe emission region comprising a biofilm) thus results in dust-mite related indoor allergens being removed.

Next to suppressing smells and allergies, the removal of biofilms according to embodiments of this invention, also have also great importance for public health; because pathogens such as e-coli, which may be hiding within the biofilms from the disinfectants contained in commonly-used cleaning detergents, may similarly be eliminated.

The system further comprises a light generating device. The term "light generating device" may especially refer to a device configured to provide (visible) light. In embodiments, the light generating device may be selected from the group of a lamp, a luminaire, a projector device, and a (UV and/or IR) disinfection device.

Hence, the system is a lighting system.

The light generating device is especially configured to provide a first beam of first device radiation. The first device radiation has a spectral power distribution selected for promoting persistence of the first microbes, especially relative to second microbes, other than the first microbes.

The term "persistence" may herein especially refer to the continued presence of the first microbes, especially relative to the second microbes. Hence, the first device radiation may have a spectral power distribution selected for promoting the presence of the first microbes, especially relative to the second microbes. In particular, in embodiments, the first device radiation may have a spectral power distribution selected for positively affecting the first microbes, such as promoting growth of the first microbes, especially relative to the second microbes. Thereby, the first microbes may accumulate in the indoor environment, such as on a surface, or such as in a space. In further embodiments, the first device radiation may have a spectral power distribution selected for negatively affecting the second microbes, such as deactivating the second microbes, especially diminishing growth of the second microbes. Thereby, the second microbes may diminish in the microbiome accumulate in the indoor environment, such as on a surface, or such as in a space, which may diminish, especially remove, a competitor for the first microbes.

In particular, the first device radiation has a spectral power distribution selected to provide a competitive advantage to the first microbes relative to the second microbes, i.e., to promote the persistence of the first microbes relative to the second microbes. A competitive advantage may be provided by providing a larger benefit (or smaller detriment) to the first microbes relative to the second microbes. Thereby, the first microbes may persist in the indoor environment, especially (over time) become more prevalent in the indoor environment, with respect to the second microbes.

Hence, in embodiments, the first device radiation may have a spectral power distribution selected to deactivate second microbes more strongly than the first microbes.

In further embodiments, the spectral power distribution of the first device radiation may be selected for one or more of (i) promoting growth of the first microbes, (ii) deactivating (or "diminishing growth of the") second microbes, other than the first microbes, (iii) deactivating second microbes more strongly than the first microbes, and (iv) deactivating viruses.

The term "second microbes" may herein refer to microbes other than the first microbes. Specifically, the first microbes may be desirable microbes in the indoor space, whereas the second microbes may be undesirable due to their effect on humans and/or on the first microbes.

Hence, the first device radiation (or the disinfection radiation; see below) may be configured to remove (or "deactivate" or "kill") a virus. **In** embodiments, the virus may comprise a human pathogen, such as an influenza or a corona virus. In further embodiments, the virus may be a microbial pathogen, such as a virus infecting the first microbes. For instance, in embodiments, the virus may comprise a bacteriophage, such as a bacteriophage targeting one or more of the first microbes.

For example, light may promote the growth of microbes, especially also of heterotrophic microbes, such as described in Fahimipour et al., "Daylight exposure modulates bacterial communities associated with household dust", Microbiome, 2018, which is hereby herein incorporated by reference, which may specifically describe that while daylight may usually be associated with the loss of microbes, it may also lead to increases in the abundances of some specific microbes. The paper may further demonstrate that light exposure per se led to lower abundances of viable bacteria and communities that were compositionally distinct from dark rooms, suggesting preferential inactivation of some microbes over others under daylighting conditions. Hence, light may be used to (i) promote growth, (ii) cause loss of microbes, and (iii) to cause more substantial loss for second microbes with respect to first microbes. Further, Maresca et al., "Light Modulates the Physiology of Nonphototrophic *Actinobacteria",* Journal of Bacteriology, 2019, which is hereby herein incorporated by reference, may describe that Actinobacteria, specifically strains *Rhodoluna lacicola* MWH-Ta8 and *Aurantimicrobium* sp. MWH-Uga, grew faster in blue light and UV light, but not in red or green light. Hence, the light exposure may have promoted the growth of these Actinobacteria.

In embodiments, the system, especially the control system (see below), may have a microbic lighting mode. Especially, in the microbic lighting mode, the light generating device may be configured to provide the first beam of the first device radiation. In particular, in the microbic lighting mode, the spectral power distribution of the first device radiation may be selected for providing a competitive advantage to the first microbe(s) relative to second microbe(s), other than the first microbe(s).

In particular, according to the invention, the microbe emission region and the first beam may at least partly spatially overlap. For instance, the microbe emission region may comprise a surface and the first beam may illuminate at least part of the surface, or the microbe emission region may comprise a (3D) space and the first beam may pass through at least part of the space. Hence, in embodiments, the microbe emission region and the first beam may (at least partially) spatially overlap at a distance from the microbe emission device and/or the light generating device, especially from the microbe emission device, or especially from the light generating device, such as at a distance selected from the range of 0.5 - 10 m.

As partly mentioned, the microbe emission region may comprise a first surface comprising a biofilm. The microbe emission device may be arranged to provide the emission of first microbes to a first surface comprising a biofilm, for example a ceiling or a wall with a biofilm layer.

In particular, the first beam may be divergent (with respect to the light generating device), and may thus cover a large area at a larger distance from the light generating device. Hence, in embodiments, the microbe emission region and the first beam may have a shared region, i.e., they overlap in the shared region, wherein the shared region, such as a shared volume, or such as a shared surface, may have a frustum-shape.

In further embodiments, the light generating device may be configured to (during the microbic lighting mode) provide the first device radiation, especially the first beam, to at least part of the microbe emission region.

The first beam and the microbe emission region may, in embodiments, spatially overlap for at least 30% of the microbe emission region, especially at least 50%, such as at least 70%, including 100%, especially with regards to an area of a surface, or especially with regards to a volume of a space.

In further embodiments, the emission of first microbes and the providing of the first beam may also temporally overlap (also see below). The system, especially the control system, may have an operational mode, especially a microbic application mode, and especially a microbic lighting mode. The term "operational mode" may also be indicated as "controlling mode". The system, or apparatus, or device (see further also below) may execute an action in a "mode" or "operational mode" or "mode of operation". Likewise, in a method an action, stage, or step may be executed in a "mode" or "operation mode" or "mode of operation". This does not exclude that the system, or apparatus, or device may also be adapted for providing another operational mode, or a plurality of other operational modes. Likewise, this does not exclude that before executing the mode and/or after executing the mode one or more other modes may be executed. However, in embodiments a control system (see further also below) may be available, that is adapted to provide at least the operational mode. Would other modes be available, the choice of such modes may especially be executed via a user interface, though other options, like executing a mode in dependence of a sensor signal or a (time) scheme, may also be possible. The operational mode may in embodiments also refer to a system, or apparatus, or device, that can only operate in a single operation mode (i.e. "on", without further tunability).

In embodiments, the microbic lighting mode and the microbic application mode may temporally overlap, i.e., the microbe dispenser device may provide an emission of first microbes to the microbe emission region while the light generating device provides the first beam of first device radiation.

In further embodiments, the microbic lighting mode be temporally arranged after the microbic application mode. Hence, the microbe dispenser device may (first) provide the first microbes to the microbe emission region, and the light generating device may subsequently provide the first beam of the first device radiation, especially to at least part of the microbe emission region. Such embodiments may be particularly beneficial when the first device radiation has a spectral power distribution selected for promoting the growth of the first microbes.

Hence, in embodiments, the microbic lighting mode may overlap in time with the microbic application mode or may be subsequent in time to the microbic application mode.

The light generating device may especially also serve as (normal) room lighting, such as office lighting. Hence, the light generating device may have a dual function, both providing lighting for normal use of the room, and providing the first device radiation for cultivating a desired microbiome in the room.

Hence, in embodiments, the system, especially the control system, may have a standard lighting mode. In further embodiments, in the standard lighting mode the light generating device may be configured to provide (standard) white (first) device radiation, especially to provide (standard) white light. In particular, the standard lighting mode may comprise providing general lighting, spot lighting, and wall washing. The term "standard lighting" may herein especially refer to lighting devoid of enriched light used to selectively promote desirable microbes.

The first device radiation provided during the microbic lighting mode may, in embodiments, also be suitable for normal indoor use. Specifically, in further embodiments, the light generating device is configured to provide in the microbic lighting mode (microbic) white first device radiation, especially (microbic) white light.

However, during the microbic lighting mode the spectral power distribution may be tailored for the cultivation of a desired indoor microbiome. Hence, in further embodiments, a relative spectral power distribution of a first wavelength range of 405 nm +/-5 nm, relative to the spectral power distribution in the wavelength range of 200-780 nm, especially 380-780 nm, such as 380 - 750 nm, may be higher during at least part of the microbic lighting mode than during at least part of the standard lighting mode, especially at least 30% higher, such as at least 60% higher, especially at least 100% higher.

Hence, in embodiments, the light provided by the light generating device may be white both in the microbic lighting mode AND in the standard lighting mode, but the first device radiation in the microbic lighting mode may be enriched in the first wavelength range compared to the spectral power distribution in the standard lighting mode.

In further embodiments, a relative spectral power distribution of a first wavelength range of 180 - 220 nm, especially 200 - 220 nm, relative to the spectral power distribution in the wavelength range of 180-780 nm, especially 180-400 nm, may be higher during at least part of the microbic lighting mode than during at least part of the standard lighting mode, especially at least 30% higher, such as at least 60% higher, especially at least 100% higher.

Similarly, in further embodiments, a relative spectral power distribution of a first wavelength range of 222 nm +/- 5 nm, relative to the spectral power distribution in the wavelength range of 200-780 nm, especially 200-400 nm, may be higher during at least part of the microbic lighting mode than during at least part of the standard lighting mode, especially at least 30% higher, such as at least 60% higher, especially at least 100% higher.

In further embodiments, a relative spectral power distribution of a first wavelength range of 270 nm +/- 10 nm, relative to the spectral power distribution in the wavelength range of 200-780 nm, especially 200-400 nm, may be higher during at least part of the microbic lighting mode than during at least part of the standard lighting mode, especially at least 30% higher, such as at least 60% higher, especially at least 100% higher.

In embodiments, the first wavelength range may have a central wavelength λ_{c} and a spread λ_{S}, such that the first wavelength range comprises the wavelength range of λ_{c}-λ_{S} - λ_{c}+λ_{S}. In particular, in such embodiments, a relative spectral power distribution of the first wavelength range, relative to the spectral power distribution in the wavelength range of 200-780 nm, such as in the wavelength range of 200-750 nm, may be higher during at least part of the microbic lighting mode than during at least part of the standard lighting mode, especially at least 30% higher, such as at least 60% higher, especially at least 100% higher. In embodiments, the spread λ_{S} may be ≤ 10 nm, such as ≤ 5 nm, especially ≤ 2.5 nm, such as ≤ 1 nm. In further embodiments, the spread λ_{S} may be ≥ 0.2 nm, such as ≥ 0.5 nm, especially ≥ 1 nm. For instance, in embodiments 0.2 nm ≤ λ_{S} ≤ 10 nm, such as 1 nm ≤ λ_{S} ≤ 5 nm. In further embodiments, central wavelength λ_{c} may be selected from the group comprising 210 nm, 222 nm, 230 nm, 254 nm, 270 nm, and 405 nm.

Hence, in embodiments, λ_{c} may be 210 nm, especially wherein λ_{S} ≤ 10 nm, such as ≤ 5 nm, especially ≤ 2.5 nm. In further embodiments, λ_{c} may be 222 nm, and λ_{S} may be ≤ 5 nm, such as 5 nm. In further embodiments, λ_{c} may be 230 nm, especially wherein λ_{S} ≤ 10 nm, such as ≤ 5 nm, especially ≤ 2.5 nm. In further embodiments, λ_{c} may be 254 nm, especially wherein λ_{S} ≤ 10 nm, such as ≤ 5 nm, especially ≤ 2.5 nm. In further embodiments, λ_{c} may be 270 nm, and λ_{S} may be ≤ 10 nm, such as 10 nm. In further embodiments, λ_{c} may be 405 nm, and λ_{S} may be ≤ 5 nm, such as 5 nm. Hence, in embodiments, a relative spectral power distribution of a first wavelength range of 405 nm +/- 5 nm, relative to the spectral power distribution in the wavelength range of 200-780 nm, especially 380-780 nm, such as 380 - 750 nm, may be higher during at least part of the microbic lighting mode than during at least part of the standard lighting mode, especially at least 30% higher, such as at least 60% higher, especially at least 100% higher.

Some wavelengths and wavelength ranges may be particularly suitable for promoting the persistence of the first microbes, especially with respect to the second microbes. In particular, these wavelengths or wavelength ranges may (substantially) differentially affect different microbes (also see below).

Hence, in further embodiments, the microbic lighting mode may comprise providing first device radiation having a wavelength selected from the range of < 240 nm, such as from the range of 100 - 240 nm, especially from the range of 180 - 240 nm. In further embodiments, the microbic lighting mode may comprise providing first device radiation having a wavelength selected from the range of 250 - 280 nm, especially from the range of 260-280 nm. In particular, the relative spectral sensitivity of viruses and bacteria may often strongly depend on the UV wavelength. The differences in relative spectral sensitivity between the different microbes at a given wavelength may generally be most pronounced in the 260 nm-280 nm range and for UV wavelengths below 240 nm whereas the spectral sensitivity delta between the microbes keeps increasing with the lower the UV wavelength is.

A desired differential effect of lighting on different microbes, especially between the first microbes and the second microbes, may be obtained at a specific wavelength. Hence, in further embodiments, the microbic lighting mode may comprise providing first device radiation having a (narrow) peak wavelength λ_{P}, especially wherein at least 30 % of the spectral power in the wavelength range of λ_{P}-5 - λ_{P}+5 nm falls within a peak wavelength range, such as at least 50%, especially at least 70%. In further embodiments, the peak wavelength range may especially comprise the range of λ_{P}-1 - λ_{P}+1 nm, such as the range of λ_{P}-0.5 - λ_{P}+0.5 nm. For instance, in embodiments, 70% of the spectral power of the first device radiation in the wavelength range of 400 - 410 nm may fall in the range of 404 - 406 nm, especially in the range of 404.5 - 405.5 nm.

The system, especially the control system may further have a disinfection mode. In the disinfection mode, the light generating device may (be configured to) provide disinfection radiation. In embodiments, the disinfection radiation may comprise UV radiation. In further embodiments, the disinfection radiation may comprise visible near UV radiation, especially comprising a disinfection wavelength selected from the range of 380 - 450 nm, such as from the range of 400 - 410 nm, especially of (about) 405 nm. In further embodiments, the disinfection radiation may comprise IR radiation, especially comprising an IR wavelength selected from the range of 750 - 2000 nm, such as from the range of 750-1500 nm. In further embodiments, the IR wavelength may be ≤ 900 nm, such as selected from the range of 750 - 900 nm. In yet further embodiments, the IR wavelength may be ≥780 nm, even more especially ≥ 900 nm, such as selected from the range of 900 - 2000 nm, especially from the range of 900 - 1500 nm, such as from the range of 900 - 1100 nm. In particular, in the disinfection mode, the light generating device may (be configured to) provide a disinfection beam of disinfection radiation, wherein the disinfection beam at least partially overlaps with the microbe emission region. Hence, in embodiments, in the disinfection mode, the light generating device may (be configured to) provide disinfection radiation to the microbe emission region.

The term visible near UV radiation may herein refer to (disinfection) radiation in the visible spectrum but close to the UV spectrum. In particular, in embodiments, the visible near UV radiation may comprise the wavelength range of 380 - 495 nm, such as the wavelength range of 380 - 450 nm, especially the wavelength range of 380 - 420 nm.

In embodiments, the control system may be configured to control the light generating device. In further embodiments, the control system may be configured to control the microbe dispenser device. In particular, the control system may be configured to control one or more of the first device radiation, the disinfection radiation, and the emission of the first microbes, especially in dependence of one or more of a sensor, a timer, a user interface, and a predetermined program. In embodiments, the lighting system, or particularly the control system may comprise such a sensor.

For instance, in embodiments, the control system may be configured to control the one or more of the first device radiation, the disinfection radiation, and the emission of the first microbes based on the sensor, especially based on a sensor signal from a sensor, especially a sensor selected from the group comprising a movement sensor, a presence sensor, an activity detection sensor, a people counting sensor, a distance sensor, an ion sensor, a gas sensor, a volatile organic compound sensor, a pathogen sensor, an airflow sensor, a sound sensor, a temperature sensor, and a humidity sensor, For example, in embodiments, the sensor may comprise a pathogen sensor, and the sensor signal may indicate a high level of an (undesirable) second microbe, which may cause the control system to initiate one or more of the first device radiation, the disinfection radiation, and the emission of the first microbes, especially the disinfection radiation. In further embodiments, the sensor may comprise a temperature sensor, and the sensor signal may indicate that the temperature is rising, which may provide a competitive benefit to the first microbes or, alternatively, to (part of) the second microbes. Hence, the control system may be configured to respond to a changing temperature by, for instance, increasing the emission of first microbes and/or increasing the first device radiation. In further embodiments, the sensor may comprise an activity sensor configured to detect (an) activity in an indoor space, such as a cleaning activity, or such as a conference meeting. In such embodiments, the control system may, for example, increase the microbe emission after the detected activity, may modulate, especially stop, disinfection radiation during the activity, and may increase wavelengths in the first device radiation that contribute to vitamin D generation (see below). Further, if the system further comprises a pathogen sensor, the system may be configured to determine a pathogen status in the indoor space after the activity to re-assess the need for subsequent disinfection. In further embodiments, the sensor may comprise a presence sensor, and the sensor signal may also indicate that a human has entered the room, which may cause the control system to modulate the disinfection radiation, especially stop the disinfection radiation, or especially alter the wavelength of the disinfection radiation, as UV radiation may also be harmful to humans. For instance, the table below may indicate properties of different disinfection wavelength ranges, including disinfection efficiency for different types of micro-organisms, and safety (to humans):

| Name | Short name | Wavelength (nm) | (Relative) sterilization effectiveness | | Safe Radiation | Vitamin D generation | Ozone generation |
|---|---|---|---|---|---|---|---|
| | | | Bacteria | Viruses | | | |
| Near Infrared | NIR | 750-950 | + | +/- | +++ | | |
| Violet | V | 380-420 | +/- | - | + | | |
| Ultra-violet A | UV-A | 315-380 | + | - | + | | |
| Ultra-violet B | UV-B | 280-315 | + | +/- | +/- | + | |
| Near ultra-violet C | Near UV-C | 230-280 | ++ | ++ | - | | |
| Far ultra-violet C | Far UV-C | 190-230 | +++ | +++ | + | | +/- |
| Extreme ultra-violet C | Extrem e UV-C | 100-190 | +++ | +++ | - | | + |

Even further, regarding the problems of biofilms mentioned before, said sensor may be a biofilm sensor, said biofilm sensor may be configured to detect a biofilm layer on a first surface, or configured to derive a presence of a biofilm (in the microbe emission region). Thereby, the control system according to the invention may be configured to control the one or more of the first device radiation, the disinfection radiation, and the emission of the first microbes based on the sensor. Said biofilm sensor may e.g. be a camera, or even a VOC sensor detecting undesired associated odors originating from a biofilm layer.

More specifically: the invention provides a lighting system for indoor microbiome management, wherein the lighting system comprises a light generating device and a microbe dispenser device; wherein the microbe dispenser device is configured to provide in a microbic application mode an emission of first microbes, wherein the microbe dispenser device has a microbe emission region; the light generating device is configured to provide in a microbic lighting mode a first beam of first device radiation; a spectral power distribution of the first device radiation is selected for promoting persistence of the first microbes relative to second microbes, other than the first microbes; and the microbe emission region and the first beam at least partly spatially overlap; and wherein, in aspects thereof, the microbe emission region comprises a first surface, wherein the first surface comprises a biofilm that is comprising said second microbes. In aspects thereof, the microbe dispenser device may be directed to said first surface, wherein said microbe emission region at least partly comprises, or covers, or overlaps, said first surface. In further aspects, the lighting system may comprise a biofilm sensor configured to sense the presence of a biofilm and/or sense said first surface comprising the biofilm. For example, the control system may comprise said biofilm sensor. The lighting system may be arranged such, that the microbe dispenser device provides said emission of first microbes to said first surface, for example when the lighting system determines that said first surface comprises a biofilm exceeding particular thresholds.

Hence, in these aspects of the invention, the lighting system is configured to (automatically) sense the presence of a biofilm, or a first surface comprising said biofilm. The presence of the biofilm may for example be: (A) directly detected by a biofilm sensor, (B) indirectly detected based on sensing odors generated by (or associated with) the presence of the biofilm, (C) derived from human reports about dust mite allergic symptoms, (D) or may be inferred by the recent activity pattern in the space such as cooking, cleaning, consumption of food and/or physical exercise involving sweating.

As partly mentioned, the microbe dispenser device will emission a probiotic layer of the first microbes into said microbe emission region and onto said first surface comprising the biofilm, the biofilm comprising said second microbes. The first beneficial microbes are capable to destruct ("eat") the detected biofilm. The first device radiation will aid and promote said first microbes therewith.

In embodiments, considering the above, the lighting system may comprise a control system, wherein the lighting system may comprise a VOC sensor configured to sense an odor, wherein the odor comprises a concentration level, wherein the control system is configured to determine a (odor) condition in which the concentration level of the sensed odor exceeds a predetermined (odor concentration level) threshold, and upon determining said (odor) condition control the microbe dispenser device to provide the emission of first microbes. Said condition may set the microbic application mode.

In further aspects, the control system may be configured to obtain a cleaning schedule associated with the microbe emission region and determine a cleaning rate associated with the microbe emission region, wherein the control system is configured to determine a (cleaning) condition in which the cleaning rate is lower than a predetermined (cleaning rate) threshold, and upon determining said (cleaning) condition control the microbe dispenser device to provide the emission of first microbes. Said cleaning rate may mean the number of instances cleaning occurs within a certain time interval, e.g. once a day, or once a week. This may be an indication of how much biofilm may be accumulated.

In yet further aspects, the lighting system may comprise a sensor configured to detect dust-mite allergens, wherein the control system is configured to determine a (allergen) condition in which a concentration of dust-mite allergens exceeds a predetermined (allergen) threshold, and upon determining said (allergen) condition control the microbe dispenser device to provide the emission of first microbes.

The use of UV for disinfection may be well-established. Also IR light may also have a disinfectant effect. In general, the term infrared refers to wavelengths above 750 nm, of which near infrared (NIR) describes wavelengths 750 - 1400 nm, of which the range of 750 - 950 nm may be particularly suitable for IR disinfection. An advantage of using IR light as disinfection light is that it may work better than UV light on certain surfaces and for certain microbes. For example, it may be well-known that NIR disinfection (750-950 nm) achieves an 80% to 99.9% or 2-3 log reduction of iron-dependent and some other types of bacteria and fungi. Furthermore, the absorption spectrum of each microbe may show some distinct absorption peaks. Consequently, specific IR wavelengths can be selected so as to 'excite' specific bond types in the specific molecules in, for instance, targeted second microbes.

Moreover, in practical disinfection lighting conditions, the target surface may be partially blocked. While a certain material may be transparent at IR frequencies (e.g. thin plastic), it may not be transparent for 405nm or UV disinfection light. It is known that infrared light can pass through many materials which visible or UV light cannot pass through. However, the reverse is also true. There are some materials such as glass which can pass visible 405nm light but not infrared.

For instance, Far Infrared rays can penetrate into the human body tissue up to 3.81 to 7.11 centimeters (1.5 to 2.8 inches) while UV light may generally be absorbed in the outer dead skin layer. Similarly, if a sheet of paper lies on a desk surface, IR may be scattered by the paper, but some of the IR light may nevertheless pass through the paper and reach the surface underneath.

In addition, additional human-centric synergetic effects between IR and UV disinfection light sources, or other dual-functions of either the IR or UV, may be realized. For instance, the IR light source may further be used to provide warmth, according to a different setting, for heating up the floor of the changing room in a shared space such as a gym or in a residential bathroom. The IR light also may be used to provide wellness feeling by heating bodies in a room directly without heating up the air. Absorbing the heat into the skin, which feels like warm sun rays on a spring day may be particularly relaxing and incredibly comforting.

As an additional advantage, the IR light may be used to prevent mold or mildew. In many spaces in wet rooms, silicone sealing gets attacked by mildew and will ask for special treatment. The IR light may be used to heat up and dry the walls (instead of the air like conventional heaters) and hence provide a mold prevention function in times of high humidity (e.g. right after shower).

It appears that the combination of UV and IR light sources may create a synergistic effect for killing pathogens. However, IR disinfection may also have drawbacks: if a high IR dose is administered to an object, it may heat to a high temperature, which may damage the object. It may hence be advantageous that a to-be-disinfected surface is covered by water film when applying the IR disinfection.

In further embodiments, the control system may be configured to control the one or more of the first device radiation, the disinfection radiation, and the emission of the first microbes based on the timer. For instance, the control system may be configured to (have the microbe dispenser device) provide a microbe emission at least every hour. Hence, if an hour has passed since a last (scheduled or spontaneous) microbe emission, the control system may (have the microbe dispenser device) provide a microbe emission of first microbes.

In further embodiments, the control system may be configured to control the one or more of the first device radiation, the disinfection radiation, and the emission of the first microbes based on the user interface. In particular, the control system may comprise the user interface. A user may, for instance, provide input to the control system to initiate or cease an operation.

In further developments, the control system may be configured to control the one or more of the first device radiation, the disinfection radiation, and the emission of the first microbes based on the predetermined program. For instance, the control system may be configured to cultivate the microbiome such that a desired microbiome is present in the morning when a user starts to use an indoor space. Hence, in embodiments, the control system may be configured to employ the one or more of the first device radiation, the disinfection radiation, and the emission of the first microbes during (at least part of) the night for indoor microbiome management. In embodiments, the control system may be configured to dynamically adapt the predetermined program based on a schedule, such as based on a calendar. Hence, the control system may be configured to retrieve (booking) information from a calendar. For instance, if a meeting room is scheduled to host a meeting at 2 PM, then the control system may control one or more of the first device radiation, the disinfection radiation, and the emission of the first microbes to provide a desired microbiome in the meeting room by 2 PM. In further embodiments, the control system may be configured to predict activities in the indoor space, such as based on historic data related to past activities, and adapt the predetermined program accordingly. For instance, a cafeteria in an office building may generally not be formally booked in a calendar, but may consistently be occupied around lunch time, which may be noted and taken into account by the control system.

In further embodiments, the control system may have access to an external database, especially wherein the external database comprises information on the first microbes and/or the second microbes. In particular, the control system may be configured to retrieve information on the first microbes and/or the second microbes from the external database, especially wherein the information comprises data on spectral sensitivities of the first microbes and/or the second microbes. In embodiments, the control system may be configured to retrieve information on the (geographic) prevalence of second microbes from the external database, such as based on a GPS location of the indoor space. Hence, the control system may be configured to determine the (likely) presence of second microbes based on information retrieved from the external database, and may select the spectral power distribution of the first device radiation based on the presence of the second microbes.

Similarly, in embodiments, the control system may comprise or be functionally coupled to a user interface. The user interface may be configured for receiving input and providing the input to the control system. In embodiments, the user interface may be configured for receiving input related to the spectral sensitivities of the first microbes and/or the second microbes. In further embodiments, the user interface may be configured for receiving input related to the type of second microbes present in the indoor space.

The disinfection radiation may also impair the first microbes, i.e., the first microbes may also be vulnerable to the disinfection radiation. Hence, in embodiments, the control system may be configured to control a microbe emission rate of the (emission of the) first microbes in dependence of the disinfection radiation.

In particular, in further embodiments, the microbic application mode and the disinfection mode may at least partly overlap in time, especially wherein relative to a baseline emission rate, such as a daily average microbe emission rate, the microbe emission rate during at least part of the disinfection mode may be higher than the baseline emission rate. Hence, as the first microbes are being inactivated/killed due to the disinfection radiation, the microbe dispenser device may simultaneously dispense the first microbes to maintain a desired balance. In particular, during periods when the disinfection radiation is actuated, extra first microbes may be actively dispensed into the indoor space to compensate for the effect of the UV light unintentionally deactivating some of the wanted biome on the surfaces. Hence, the microbe dispenser device may concurrently replenish those good bacteria, which got unintentionally deactivated by the disinfection radiation.

The term indoor space may for instance relate to a (part of) hospitality area, such as a restaurant, a hotel, a clinic, or a hospital, etc.. The term "space" may also relate to (a part of) an office, a department store, a warehouse, a cinema, a church, a theatre, a library, etc. However, the term "space" also relate to (a part of) a working space in a vehicle, such as a cabin of a truck, a cabin of an air plane, a cabin of a vessel (ship), a cabin of a car, a cabin of a crane, a cabin of an engineering vehicle like a tractor, etc.. The term "space" may also relate to (a part of) a working space, such as an office, a (production) plant, a power plant (like a nuclear power plant, a gas power plant, a coal power plant, etc.), etc. For instance, the term "space" may also relate to a control room, a security room, etc.. Especially, the term "space" may herein refer to an indoor space. In yet other embodiments, the term "space" may also relate to a toilet room or bathroom. In yet other embodiments, the term "space" may also relate to an elevator. In embodiments, the term "space" may also refer to a conference room, a school room, an indoor hallway, an indoor corridor, an indoor space in an elderly home, an indoor space in a nursing home, etc. In embodiments, the term "space" may refer to an indoor sport space, like a gym, a gymnastics hall, in indoor ball sport space, a ballet room, a swimming pool, a changing room, etc. In embodiments, the term "space" may refer to an (indoor) bar, an (indoor) disco, etc.

In further embodiments, the microbic application mode and the disinfection mode may be temporally separated, i.e., the microbic application mode and the disinfection mode do not overlap in time. In such embodiments, relative to the baseline microbe emission rate, the microbe emission rate after the disinfection mode may be higher than the baseline microbe emission rate. Hence, after the disinfection radiation has disinfected a space/region, especially the microbe emission region, the space/region may be repopulated with the first microbes.

Hence, the disinfection mode may especially be used to cleanse (or "prepare") the microbe emission region for the first microbes. In particular, the disinfection radiation may be used to remove (or "deactivate" or "kill") second microbes or viruses inhabiting (at least part of) the microbe emission region, thereby reducing the competition for the first microbes in the microbe emission region. Hence, the disinfection mode may facilitate the first microbes persisting on the microbe emission region. In further embodiments, the disinfection mode may (thus) be temporally arranged (directly) before the microbic application mode.

In further embodiments, the control system may be configured to select the spectral power distribution of the first device radiation to (a) promote the persistence, especially growth, of the first microbes (relative to the second microbes), and (b) to deactivate a virus.

The term "baseline emission rate" may herein refer to a default microbe emission rate. In embodiments, the microbe emission rate may be (essentially) constant over time. In further embodiments, the microbe emission rate may follow a temporal pattern. In further embodiments, the microbe emission rate may be irregular over time. In further embodiments, the baseline emission rate may be (essentially) 0, and the microbic application mode may be initiated (only) when specific conditions apply, such as when user input is received, and/or such as (directly) after the disinfection mode.

In further embodiments, the baseline emission rate may be a daily average emission rate.

In further embodiments, the control system may be configured to control the first device radiation, the disinfection radiation, and the microbe emission of the first microbes in dependence of information from a room context awareness system. In particular, the room context awareness system may detect whether a human is present in the room and provide a related presence signal to the control system, potentially classifying the person (e.g. elderly vs young person, whom may have different sensitivities to different wavelengths). Hence, the room context awareness system may especially comprise one or more of a movement sensor, a presence sensor, and a people counting sensor. In embodiments, the control system may control the microbe dispenser device based on both operation status of the disinfection mode and the room-occupancy. For instance, the microbes may preferably be dispersed when the room is known to remain unoccupied for a certain time period, so that after dispensing of the aerosolized microbes, they can sufficiently settle and multiply on surfaces in the indoor space. Once an occupant enters the room, for instance in the next morning, the concentration of aerosolized microbes may have increased below a threshold value, while the surfaces have a high microbe concentration. In addition, the lighting generating system may be adjusted during the night to promote maximum growth of the dispensed first microbes.

The term "X-related signal", such as the presence related signal, may herein refer to a signal that is related to the detected X, such as to the detected presence. In particular, the X-related signal may comprise raw and/or processed data related to the (detected) X. Hence, the related presence signal may especially comprise raw and/or processed data related to the presence of a human in the room.

The microbe dispenser device may be beneficially configured for improving room comfort. For instance, in embodiments, the microbe dispenser device may comprise a mist dispenser, wherein the mist dispenser may be configured to dispense the first microbes. In particular, the mist dispenser may further be configured to provide mist, which may form part of a calming (office) atmosphere.

In further embodiments, the microbe dispenser device may be configured to (also) dispense one or more odors. Hence, the microbe dispenser device may simultaneously provide a healthier indoor environment by dispensing the first microbes, and provide a more pleasant indoor environment by dispensing the odor, which may also enhance user awareness to the cleansing effects of the microbe dispenser device.

In particular, in embodiments, the microbe dispenser device may comprise a cartridge holder. The cartridge holder may especially be configured to detachably host one or more cartridges, especially a plurality of cartridges. In embodiments, the cartridge holder may be configured to host at least a cartridge comprising (at least part of) the first microbes. In further embodiments, the cartridge holder may be configured to host a plurality of cartridges comprising (different) first microbes. In further embodiments, the cartridge holder may be configured to host a cartridge comprising a type of odor, especially an odor compound.

In specific embodiments, the cartridge holder may be configured to host a plurality of cartridges, wherein two or more of the plurality of cartridges are filled with material differing in one or more of (a) types of first microbes and (b) types of odor.

In a further aspect, the invention may provide a (lighting) device comprising (i) the lighting system according to the invention and a housing. The housing may especially enclose at least part of the light generating device and at least part of the microbe dispenser device. The device may especially comprise a lighting device. Such housing may thus comprise the light generating device, the microbe dispenser device, and optionally, but preferably also a control system according to the invention, wherein the control system is configured to control said light generating device and the microbe dispenser device.

In embodiments, the housing may essentially enclose the light generating device. In further embodiments, the housing may essentially enclose the microbe dispenser device.

In further embodiments, the device may be configured such that a first beam of first device radiation has a first direction , and wherein a second beam of disinfection radiation has a second direction , wherein the first direction and the second direction have a mutual angle (α_{M}) selected from the range of 60-180°, especially from the range of 90-180°, such as from the range of 120-180°. In particular, in further embodiments, the device may be configured such that a first beam of first device radiation has a first direction parallel to a first optical axis (O1) (of the first beam); and wherein a second beam of disinfection radiation has a second direction parallel to a second optical axis (O2) (of the second beam), wherein the first direction and the second direction have a mutual angle (α_{M}) selected from the range of 60-180°, especially from the range of 90-180°, such as from the range of 120-180°. In particular, in such embodiments, the first device radiation and the disinfection radiation may be spatially separated. Especially, the microbe emission region may be spatially (essentially) non-overlapping with the disinfection radiation. Hence, the disinfection radiation may be provided to one part of the space, such as a ceiling, whereas the first device radiation may be provided to a different part of the space, such as to a table surface. Especially, the first direction may coincide with the first optical axis (of the first device radiation). Yet, in embodiments the second direction may coincide with the second optical axis (of the second beam of disinfection radiation).

In further embodiments, the device may especially comprise an upper air disinfection device configured to provide disinfection radiation to the ceiling and/or a (top) part of a wall. In aspects, for example, said ceiling and/or top part of a wall may be susceptible to biofilms and/or may comprise a biofilm layer.

In a further aspect, the invention provides a method for indoor microbiome management according to claim 13.

The method especially comprises providing first device radiation and an emission of first (probiotic) microbes in an indoor space. A spectral power distribution of the first device radiation is selected for promoting persistence, especially growth, of the first microbes relative to second microbes, other than the first microbes. In further embodiments, the spectral power distribution of the first device radiation may be selected for one or more of (i) promoting growth of the first microbes, (ii) deactivating second microbes, other than the first microbes, (iii) and deactivating second microbes more strongly than the first microbes.

Hence, according to the invention, the method comprises providing in an indoor space (a) first device radiation, and (b) an emission of first microbes; wherein a spectral power distribution of the first device radiation is selected for promoting persistence of the first microbes relative to second microbes, other than the first microbes.

According to the invention, the method provides a microbic lighting mode. The microbic lighting mode comprises providing a first beam of first device radiation.

According to the invention, the method provides a microbic application mode. The microbic application mode comprises providing an emission of the first microbes in a microbe emission region.

In examples, as partly mentioned before, said microbe emission region may at least partly comprise (or: cover, or: be directed to) a first surface, wherein said first surface may comprise a biofilm. Hence, the microbe emission region may comprise a region comprising a biofilm. Said biofilm may comprise said second microbes.

In further embodiments, the microbic lighting mode and the microbic application mode may temporally overlap, i.e., the microbic lighting mode may overlap in time with the microbic application mode. In further embodiments, the microbic application mode may be temporally arranged (directly) after the microbic lighting mode, i.e., the microbic lighting mode may be subsequent in time to the microbic application mode.

According to the method of the invention, the first beam and the microbe emission region at least partly spatially overlap.

The method may, in embodiments, further comprise a disinfection mode. In further embodiments, the method, especially the disinfection mode, may comprise directing at least part of disinfection radiation to an upper air space, such as to a ceiling, especially wherein the disinfection radiation comprises UV radiation. In further embodiments, the method, especially the microbic lighting mode, may comprise directing at least part of the first beam of first device radiation to a floor. In further embodiments, the method, especially the microbic application mode, may comprise directing at least part of the emission of the first microbes to the floor. In particular, in embodiments, the method may comprise providing disinfection radiation and microbic lighting radiation at a mutual angle (α_{M}) selected from the range of 60-180°, especially from the range of 90-180°, such as from the range of 120-180°.

The lighting device may be part of or may be applied in e.g. office lighting systems, household application systems, shop lighting systems, home lighting systems, accent lighting systems, spot lighting systems, theater lighting systems, fiber-optics application systems, projection systems, self-lit display systems, pixelated display systems, segmented display systems, warning sign systems, medical lighting application systems, indicator sign systems, decorative lighting systems, portable systems, automotive applications, green house lighting systems, horticulture lighting, etc.

The term white light (or "white radiation") herein, is known to the person skilled in the art. It especially relates to light having a correlated color temperature (CCT) between about 2000 and 20000 K, especially 2700-20000 K, for general lighting especially in the range of about 2700 K and 6500 K, and for backlighting purposes especially in the range of about 7000 K and 20000 K, and especially within about 15 SDCM (standard deviation of color matching) from the BBL (black body locus), especially within about 10 SDCM from the BBL, even more especially within about 5 SDCM from the BBL.

### BRIEF DESCRIPTION OF THE DRAWINGS

Embodiments of the invention will now be described, by way of example only, with reference to the accompanying schematic drawings in which corresponding reference symbols indicate corresponding parts, and in which:
Fig. 1A and 2A schematically depict embodiments of the system, and
Fig. 1C schematically depicts a spectral power distribution for the standard lighting mode, and

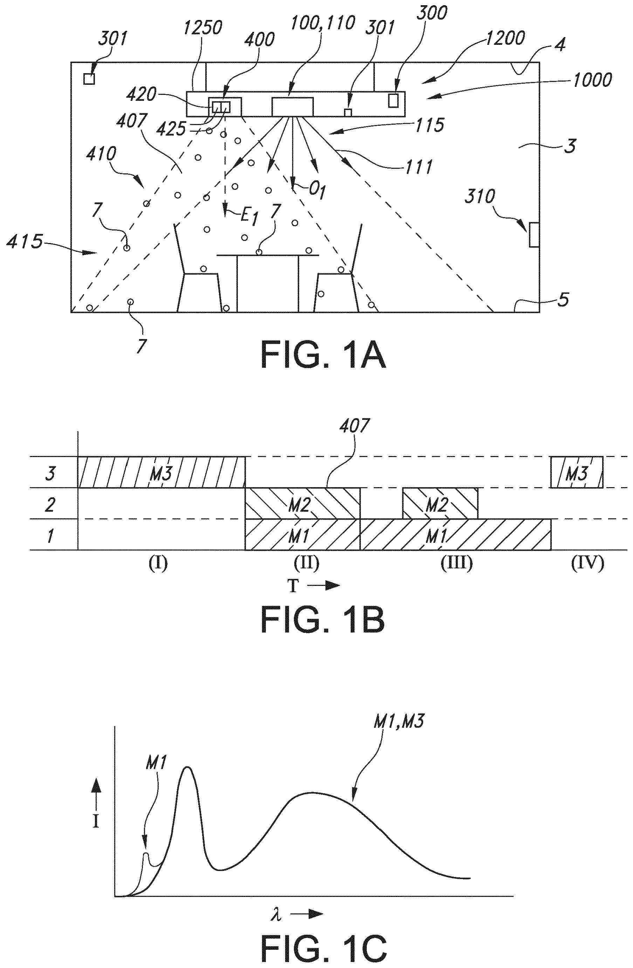

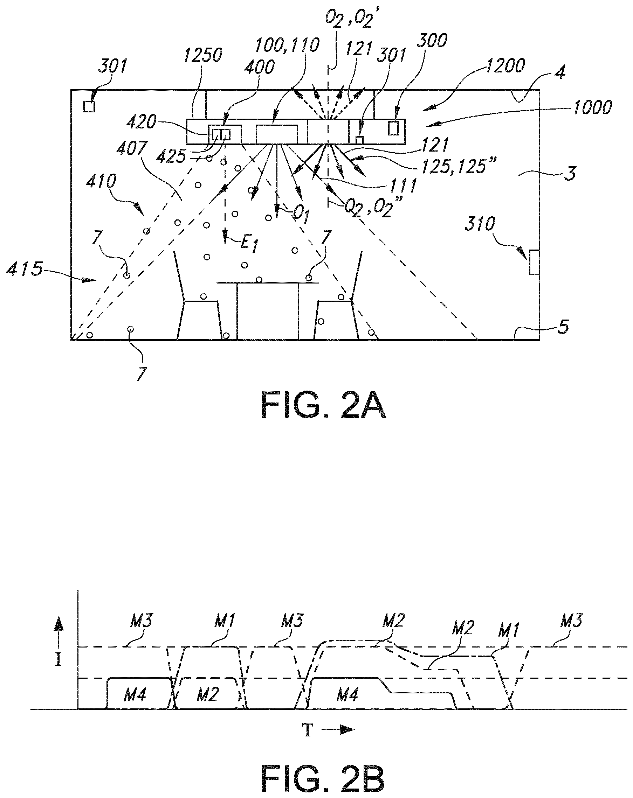

Fig. 1B and 2B schematically depict embodiments of operational modes of the system, and

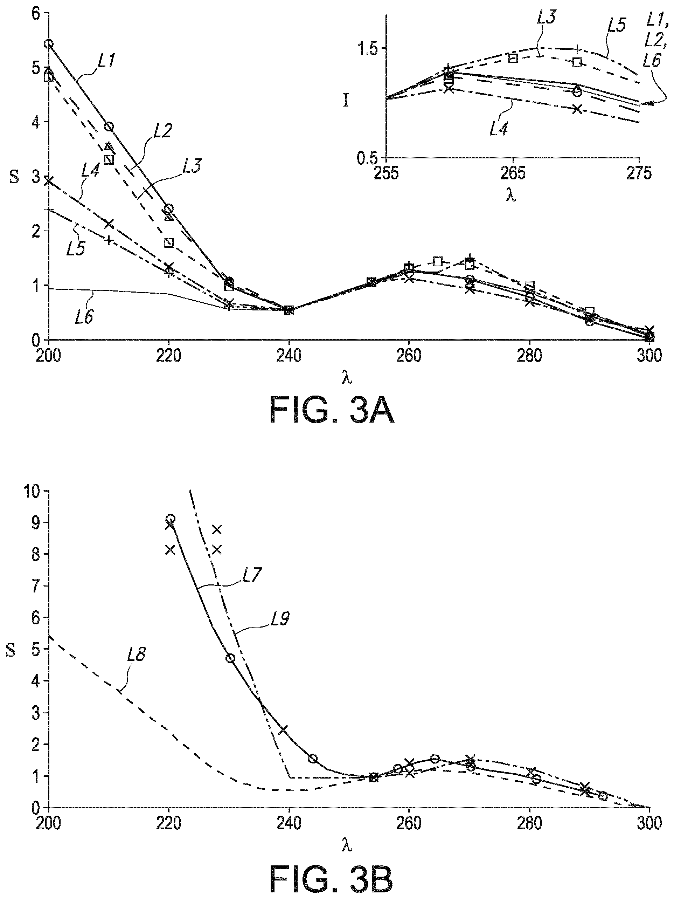

Fig. 3A-B schematically depict relative spectral sensitivities S versus wavelength λ (in nm).

The schematic drawings are not necessarily on scale.

### DETAILED DESCRIPTION OF THE EMBODIMENTS

Fig. 1A schematically depicts an embodiment of a system 1000, especially a lighting system, for indoor microbiome management. The system 1000 comprises a light generating device 100 and a microbe dispenser device 400. The system 1000 may further comprise a control system 300, which may especially be configured to control the light generating device 100, and/or the microbe dispenser device 400. In embodiments, the system 1000, especially the control system 300, may have a microbic application mode and a microbic lighting mode. In the microbic application mode, the microbe dispenser device 400 may (be configured to) provide a (microbe) emission 407 of first microbes 7, especially wherein the microbe dispenser device 400 has a microbe emission region (or range) 415, i.e., the microbe dispenser device 400 may be configured to provide an emission 407 of first microbes 7 to the microbe emission region 415. In the microbic lighting mode, the light generating device 100 may (be configured to) provide a first beam 115 of first device radiation 111. In embodiments, a spectral power distribution of the first device radiation 111 may be selected for promoting persistence, especially growth, of the first microbes 7 relative to second microbes, other than the first microbes. In further embodiments, the microbe emission region 415 and the first beam 115 may at least partly spatially overlap, such as for at least 30% of the microbe emission region 415, especially for at least 50%.

In alternative embodiments, said emission region may comprise (or: cover, or: be directed to) a first surface comprising a biofilm comprising the second microbes.

In particular, in an operational mode of the lighting system 1000 the spectral power distribution of the first device radiation 111 may be selected for providing a competitive advantage to the first microbes 7 relative to second microbes. In further embodiments, the spectral power distribution of the first device radiation 111 may be selected for one or more of (i) promoting growth of the first microbes 7, (ii) deactivating (or "eliminating") the second microbes, other than the first microbes 7, and (iii) deactivating second microbes more strongly than the first microbes 7.

In the depicted embodiment, (in the microbic application mode) the microbe dispenser device 400 may (be configured to) provide a spray 410 of the first microbes 7. Said spray may for example be directed for example to a first surface comprising a biofilm comprising the second microbes.

The microbe dispenser device 400 may provide the first microbes 7 in an average direction E₁. The average direction may be a direction obtained by determining the direction wherein in average the most first microbes propagate away from the microbe dispenser device 400. For instance, the spray direction may be the average direction in which the first microbes propagate away from a spray microbe dispenser device.

The light generating device 100 may especially be configured to provide both the first radiation during the microbic lighting mode and general lighting during a standard lighting mode. Hence, the system 1000, especially the control system 300, may further have a standard lighting mode. In embodiments, the light generating device 100 may in the standard lighting mode be configured to provide white first device radiation 111. Further, in embodiments, the light generating device 100 may be configured to provide in the microbic lighting mode white first device radiation 111. In such embodiments, a relative spectral power distribution of a first wavelength range, relative to the spectral power distribution in the wavelength range of 200-780 nm, especially 380-750 nm, or especially 200-400 nm, may be higher during at least part of the microbic lighting mode than during at least part of the standard lighting mode. In further embodiments, the first wavelength range may especially comprise the wavelength range of 405 nm +/- 5 nm. Hence, the microbic lighting mode may be enriched with wavelengths selected for microbe modulation relative to the standard lighting mode.

By default, general lighting may have little to no UV radiation. However, a wavelength in the UV range may be selected for promoting the persistence of the first microbes 7, especially relative to the second microbes. Hence, in further embodiments, a relative spectral power distribution of a first wavelength range of 222 nm +/- 5 nm, relative to the spectral power distribution in the wavelength range of 200-400 nm, may be higher during at least part of the microbic lighting mode than during at least part of the standard lighting mode.

Hence, in embodiments, the light generating device 100 may be configured to provide in a standard lighting mode white first device radiation 111, wherein the light generating device 100 is configured to provide in the microbic lighting mode white first device radiation 111; wherein a relative spectral power distribution of a first wavelength range relative to the spectral power distribution in the wavelength range of 200-780 nm, is higher during at least part of the microbic lighting mode than during at least part of the standard lighting mode. In further embodiments, the first wavelength range comprises the range of 405 nm +/- 5 nm. In further embodiments, the first wavelength range comprises the range of 222 nm +/- 5 nm.

The microbic application mode, the microbic lighting mode, and the standard lighting mode may (partially) overlap (in time).

Fig. 1B schematically depicts an embodiment of a temporal arrangement of the microbic application mode, the microbic lighting mode, and the standard lighting mode over time T, wherein M1 refers to the microbic lighting mode, M2 refers to the microbic application mode, and M3 refers to the standard lighting mode. In a first phase I, the system may be in the standard lighting mode. Subsequently, in a second phase, the system may simultaneously be in the microbic application mode and the microbic lighting mode, i.e., the microbe dispenser device 400 may provide an emission 407 of first microbes 7, and the light generating device 100 may provide a first beam 115 of first radiation 111 to promote the persistence of the first microbes 7, especially relative to second microbes. At the end of the second phase II, the microbic application mode may cease until about 1/5^{th} of the way through a third phase III, during which the microbic lighting mode is continuously active, and during which the microbic application mode is partially active. At the end of the third phase III, the microbic lighting mode may switch off, and the standard lighting mode may be started for the fourth phase IV.

For instance, phases I may correspond to the end of a (working) day, during which standard lighting is used. At the end of the (working) day, such as after people leave the indoor space 3, the second phase II may begin and the system 1000 may initiate the microbic application mode and the microbic lighting mode. During phases II and III the system 1000 may prepare the indoor space 3 for the next (working) day. As indicated in the embodiment, the microbe emission 407 of first microbes 7 may be provided multiple times. For example, the system 1000 may during phase II providing a first set of first microbes 7 and may during phase III provide a second set of first microbes 7, such as a second set of first microbes belonging to different genera than the first microbes 7 of the first set. This could, for example, be relevant if the different first microbes 7 may be beneficially cultivated with first radiation 111 having different first spectral distributions. Or, for example, if the first microbes 7 of the second set depend on the first microbes 7 of the first set, which may, in such embodiments, first be allowed to settle in the indoor space 3. As the next (working) day starts, in phase IV, the system 1000 may again switch to standard lighting.

In further embodiments, the system may also execute the microbic lighting mode while the indoor space 3 is in use (by a human user).

In the depicted embodiment, the microbic lighting mode overlaps in time with the microbic application mode. In further embodiments, the microbic lighting mode may be arranged subsequent in time to the microbic application mode.

Fig. 1C schematically depicts a spectral power distribution for the standard lighting mode M3 and for the microbic lighting mode M1 in intensity I vs. wavelength λ. In particular, in the depicted embodiment, a relative spectral power distribution of a first wavelength range relative to the spectral power distribution in a reference wavelength range, such as the wavelength range of 200-780 nm, is higher during at least part of the microbic lighting mode M1 than during at least part of the standard lighting mode M3. In particular, in the depicted embodiment, the microbic lighting mode M1 may have an additional peak in the spectral power distribution at the left side of the distribution.

Fig. 2A schematically depicts a further embodiment of the system 1000. In the depicted embodiment, the light generating device 100 may in the disinfection mode (be configured to) provide disinfection radiation 121, especially wherein the disinfection radiation 121 comprises UV radiation. In further embodiments, the control system 300 may be configured to control the first device radiation 111, the disinfection radiation 121, and the emission 407 of the first microbes 7, especially in dependence of one or more of a sensor 301, a timer, a user interface 310, and a predetermined program. Especially the sensor 301 may be selected from the group comprising a movement sensor, a presence sensor, an activity detection sensor, a people counting sensor, a distance sensor, an ion sensor, a gas sensor, a volatile organic compound sensor, a pathogen sensor, an airflow sensor, a sound sensor, a temperature sensor, and a humidity sensor.

For instance, in the depicted embodiment, the light generating device 100 may be configured to provide the first radiation 111 centered along a first optical axis O1, such as in a cone-shape centered along the first optical axis O₁, and the disinfection radiation 121 centered along a second optical axis O2. In particular, the light generating device 100 may be configured to provide the disinfection radiation centered along the second optical axis O2 in a first direction, indicated by O2' towards an upper air space, especially towards the ceiling 4, or in a second direction, indicated by O2", towards the floor 5. In further embodiments, the control system 300 may, for instance, control the disinfection radiation in the second direction in dependence of a signal from a sensor 301, such as of a presence sensor. Thereby, the system 1000, especially the control system 300, may avoid providing the disinfection radiation, especially comprising UV radiation, to a human in the indoor space 3.

In the depicted embodiment, the light generating device 100 may be configured to provide the disinfection radiation towards an upper air space, which may be safely removed from potential people in the room. For instance, the upper air space may be the space in the indoor space 3 above about 2.3 m.

Further, in the depicted embodiment, the light generating device 100 may be configured hanging from the ceiling 4. However, in further embodiments, the light generating device 100 may comprise a task light, such as a free floor standing office luminaire or a reading light on a table.

Hence, in embodiments, the control system 300 may be configured to control the first device radiation 111, the disinfection radiation 121, and the microbe emission 407 of the first microbes 7 in dependence of information from a room context awareness system.

In embodiments, the control system 300 may be configured to control a microbe emission rate of (the emission 407 of) the first microbes 7 in dependence of the disinfection radiation 121. Especially, one or more of the following may apply: (a) the microbic application mode and the disinfection mode may at least partly overlap in time, and relative to a baseline emission rate, the microbe emission rate during at least part of the disinfection mode is higher than the baseline emission rate; and/or (b) the microbic application mode and the disinfection mode may be temporally separated, and wherein relative to the baseline microbe emission rate, the microbe emission rate after the disinfection mode is higher than the baseline microbe emission rate. Hence, in embodiments, the microbe dispenser device 400 may be configured to increase a microbe emission rate during the disinfection mode in order to counteract negative effects of the disinfection radiation on the first microbes 7, or the microbe dispenser device 400 may be configured to increase a microbe emission rate after the disinfection mode in order to (re-)colonize the indoor space 3. In particular, in the latter embodiment, the microbe emission rate of the microbe dispenser device during the disinfection mode may be (essentially) 0, i.e., the microbe application mode may be temporally arranged (directly) after the disinfection mode.

In the depicted embodiments, the microbe dispenser device 400 may comprise a cartridge holder 420, especially wherein the cartridge holder 420 is configured to detachably host a plurality of cartridges 425. The plurality of cartridges may especially host (different) first microbes 7, such as to subsequently apply different first microbes as described above, and/or may comprise odors, especially odorous compounds. In further embodiments, two or more of the plurality of cartridges 425 may filled with material differing in one or more of types of first microbes 7 and types of odor.

In further embodiments, the microbe dispenser device 400 may comprise a mist dispenser, especially wherein the mist dispenser is configured to dispense the first microbes 7.

Fig. 2A further schematically depicts a lighting device 1200 comprising the lighting system 1000 according to any one of the preceding claims and a housing 1250. In particular, the housing 1250 may enclose at least part of the light generating device 100 and at least part of the microbe dispenser device 400.

In embodiments, the first beam 115 of first device radiation 111 may have a first direction V1 parallel to a first optical axis O1 of the first beam 115, and the second beam 125 of disinfection radiation 121 may have a second direction V2 parallel to a second optical axis O2 of the second beam 125, especially in a second direction indicated by O2", wherein the first direction V1 and the second direction V2 have a mutual angle αM selected from the range of 90-180°. In the depicted embodiment αM may especially be about 180°.

Fig. 2A further schematically depicts a method for indoor microbiome management, wherein the method comprises providing in an indoor space 3 first device radiation 111 and an emission 407 of first microbes 7, wherein a spectral power distribution of the first device radiation 111 is selected for promoting persistence of the first microbes 7 relative to second microbes, other than the first microbes 7. In embodiments, the method may comprise selected the spectral power distribution for one or more of (i) promoting growth of the first microbes 7, (ii) deactivating the second microbes, other than the first microbes 7, and (iii) deactivating the second microbes more strongly than the first microbes 7.

In the depicted embodiment, the method may comprise providing in a microbic lighting mode a first beam 115 of first device radiation 111, and providing in a microbic application mode an emission 407, especially a spray 410, of the first microbes 7 in a microbe emission region 415. In particular, in the depicted embodiment, the first beam 115 and the microbe emission region 415 at least partly spatially overlap. In further embodiments, the microbic lighting mode may temporally overlaps with the microbic application mode, or may be subsequent in time to the microbic application mode.

In further embodiments, the method may comprise directing in a disinfection mode at least part of disinfection radiation 121 to a ceiling 4, especially wherein the disinfection radiation 121 comprises UV radiation.

In further embodiments, the method may comprise directing in the microbic lighting mode at least part of the first beam 115 of first device radiation 111 to a floor 5, and especially directing in the microbic application mode at least part of the emission 407 of the first microbes 7 to the floor 5.

Fig. 2B schematically depicts an embodiment of the method, and an embodiment of an operational mode of the system, wherein intensity I of radiation employed in different modes is very schematically depicted over time T. In particular, M1 may refer to the microbic lighting mode, M2 refers to the microbic application mode, M3 may refer to the standard lighting mode, and M4 may refer to the disinfection mode. By way of example, several situations are depicted over time, which do not necessarily occur one after the other, but are only depicted in a single drawing by way of comparison.

The drawing starts on the left with a standard lighting mode M3, e.g. white light, but also a disinfection mode M4. Due to the latter, microbes may be treated detrimentally. No microbes are applied yet.

Then, in a second stage, the standard lighting mode M3 changes into a microbic lighting mode M1, e.g. by adding intensity in the desired spectral range. If desired, the spectral power distribution may be changed a bit further, to obtain essentially the same color point and/or CRI even though the intensity in the desired spectral range is added. About at the same time, the microbic application mode M2 starts. Hence, now the room is substantially treated in the first state to reduce microbes (first and second microbes), now desired microbes are added, together with the beneficial light. Hence, the spectral power distribution of the first device radiation is selected for promoting persistence of the first microbes relative to second microbes.

When the desired situation is reached, the microbic application mode M2 may in the next stage be terminated, and the microbic lighting mode M1 may also be changed into the standard lighting mode M3.

In the fourth, longer stage it is suggested that at the same time the microbic application mode M2 and the disinfection mode M4 is applied. Hence, while undesired second microbes may de detrimentally treated, desired first microbes may be relatively promoted. By way of example the microbic lighting mode M1 is drawn at a higher intensity level than in the second stage, just to indicate by way of example that due to the disinfection mode M4, it may be desirable to stronger promote the first microbes over the second microbes by the microbic light in the microbic lighting mode M1. Further, by way of example in this first stage, some intensity levels are changed over time. Again, when the desired situation is reached, the microbic application mode M2 may be terminated, and the microbic lighting mode M1 may be changed into the standard lighting mode M3.

Fig. 3A-B schematically depict relative spectral sensitivities S versus wavelength λ (in nm). In particular, line L1 corresponds to MS2, line L2 corresponds to QB, line L3 corresponds to T1UV, line L4 corresponds to T7m, line L5 corresponds to T7 Coliphages, line L6 corresponds to *C. parvum,* line L7 and the crosses correspond to *Bacillus pumilis,* line L8 corresponds to MS2, and line L9 corresponds to Adenovirus. Data points at 200 and 300 nm are extrapolated.

The term "spectral sensitivity" may herein especially refer to the relative absorption of photons at the given wavelength, which photons may damage the microbes. As indicated above, microbes may further differ in their ability to repair UV-induced damaged, such as via DNA-repair mechanisms. Hence, in embodiments, the control system may select the spectral power distribution, and especially also the irradiance, of the first device radiation and/or of the disinfection radiation based on spectral sensitivities and repair mechanisms of the first microbes and the second microbes.

Hence, as illustrated in Fig. 3A-B, different microbes may have different spectral sensitivities for different wavelengths, which may facilitate providing first device radiation that provides first microbes a competitive advantage over second microbes, other than the first microbes. In particular, the differences in the relative spectral sensitivity between the different microbes may be most pronounced in the 260nm-280nm range (with most difference at 270nm) and for UV wavelengths below 240nm.

The term "plurality" refers to two or more. Furthermore, the terms "a plurality of" and "a number of" may be used interchangeably.

The terms "substantially" or "essentially" herein, and similar terms, will be understood by the person skilled in the art. The terms "substantially" or "essentially" may also include embodiments with "entirely", "completely", "all", etc. Hence, in embodiments the adjective substantially or essentially may also be removed. Where applicable, the term "substantially" or the term "essentially" may also relate to 90% or higher, such as 95% or higher, especially 99% or higher, even more especially 99.5% or higher, including 100%. Moreover, the terms "about" and "approximately" may also relate to 90% or higher, such as 95% or higher, especially 99% or higher, even more especially 99.5% or higher, including 100%. For numerical values it is to be understood that the terms "substantially", "essentially", "about", and "approximately" may also relate to the range of 90% - 110%, such as 95%-105%, especially 99%-101% of the values(s) it refers to.

The term "comprise" also includes embodiments wherein the term "comprises" means "consists of".

The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of" but may in another embodiment also refer to "containing at least the defined species and optionally one or more other species".

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices, apparatus, or systems may herein amongst others be described during operation. As will be clear to the person skilled in the art, the invention is not limited to methods of operation, or devices, apparatus, or systems in operation.

The term "further embodiment" and similar terms may refer to an embodiment comprising the features of the previously discussed embodiment, but may also refer to an alternative embodiment.

It should be noted that the above-mentioned embodiments illustrate rather than limit the invention, and that those skilled in the art will be able to design many alternative embodiments without departing from the scope of the appended claims.

In the claims, any reference signs placed between parentheses shall not be construed as limiting the claim.

Use of the verb "to comprise" and its conjugations does not exclude the presence of elements or steps other than those stated in a claim. Unless the context clearly requires otherwise, throughout the description and the claims, the words "comprise", "comprising", "include", "including", "contain", "containing" and the like are to be construed in an inclusive sense as opposed to an exclusive or exhaustive sense; that is to say, in the sense of "including, but not limited to".

The article "a" or "an" preceding an element does not exclude the presence of a plurality of such elements.

The invention may be implemented by means of hardware comprising several distinct elements, and by means of a suitably programmed computer. **In** a device claim, or an apparatus claim, or a system claim, enumerating several means, several of these means may be embodied by one and the same item of hardware. The mere fact that certain measures are recited in mutually different dependent claims does not indicate that a combination of these measures cannot be used to advantage.

The invention also provides a control system that may control the device, apparatus, or system, or that may execute the herein described method or process. Yet further, the invention also provides a computer program product, when running on a computer which is functionally coupled to or comprised by the device, apparatus, or system, controls one or more controllable elements of such device, apparatus, or system.

The invention further applies to a device, apparatus, or system comprising one or more of the characterizing features described in the description and/or shown in the attached drawings. The invention further pertains to a method or process comprising one or more of the characterizing features described in the description and/or shown in the attached drawings. Moreover, if a method or an embodiment of the method is described being executed in a device, apparatus, or system, it will be understood that the device, apparatus, or system is suitable for or configured for (executing) the method or the embodiment of the method, respectively.

The various aspects discussed in this patent can be combined in order to provide additional advantages. Further, the person skilled in the art will understand that embodiments can be combined, and that also more than two embodiments can be combined. Furthermore, some of the features can form the basis for one or more divisional applications.

## Claims

1. A lighting system (1000) for indoor microbiome management, wherein the lighting system (1000) comprises a light generating device (100) and a microbe dispenser device (400); wherein:
- the microbe dispenser device (400) is configured to provide in a microbic application mode an emission (407) of first microbes (7), wherein the microbe dispenser device (400) has a microbe emission region (415);
- the light generating device (100) is configured to provide in a microbic lighting mode a first beam (115) of first device radiation (111);
- a spectral power distribution of the first device radiation (111) is selected for promoting persistence of the first microbes (7) relative to second microbes, other than the first microbes; and
- the microbe emission region (415) and the first beam (115) at least partly spatially overlap.

2. The lighting system (1000) according to claim 1, wherein the microbic lighting mode overlaps in time with the microbic application mode or is subsequent in time to the microbic application mode.

3. The lighting system (1000) according to any one of the preceding claims, wherein the spectral power distribution of the first device radiation (111) is selected for one or more of (i) promoting growth of the first microbes (7), (ii) deactivating second microbes, other than the first microbes (7), (iii) deactivating second microbes more strongly than the first microbes (7), and (iv) deactivating viruses.

4. The lighting system (1000) according to any one of the preceding claims, wherein the microbe dispenser device (400) is configured to provide in the microbic application mode a spray (410) of the first microbes (7).

5. The lighting system (1000) according to any one of the preceding claims, wherein (i) the light generating device (100) is configured to provide in a standard lighting mode white first device radiation (111), and wherein (ii) the light generating device (100) is configured to provide in the microbic lighting mode white first device radiation (111); wherein a relative spectral power distribution of a first wavelength range relative to the spectral power distribution in the wavelength range of 200-780 nm is higher during at least part of the microbic lighting mode than during at least part of the standard lighting mode, wherein:
- the first wavelength range comprises the range of 222 nm +/- 5 nm; or
- the first wavelength range comprises the range of 270 nm +/- 10 nm; or
- the first wavelength range comprises the range of 405 nm +/- 5 nm.

6. The lighting system (1000) according to any one of the preceding claims, wherein the first microbes (7) are selected from the group comprising *Akkermansia, Bifidobacterium, Lachnospira, Alistipes, Bacteroides, Coprococcus, Viellonella, Faecalibacterium, Roseburia, Dialister, Sutterella, Methanobrevibacter, Lactobacillus, Parabacteroides, Prevotella, Agathobacter, Eubacterium, Ruminococcus, Nitrosomonas, Nitrosospira, Nitrosopumilus, Cenarchaeum, Nitrosoarchaeum, Nitrosocaldus, Caldiarchaeum, Acinetobacter, Alcaligenes, Arthrobacter, Azospirillum, Azotobacter, Bacillus, Beijerinckia, Enterobacter, Erwinia, Flavobacterium, Rhizobium, Serratia* and *Deinococcus.*

7. The lighting system (1000) according to any one of the preceding claims, wherein the light generating device (100) is configured to provide in a disinfection mode disinfection radiation (121), wherein the disinfection radiation (121) comprises one or more of (i) UV radiation having one or more wavelengths selected from the wavelength range of 100 - 380 nm, (ii) visible near UV radiation having one or more wavelengths selected from the wavelength range of 380 - 495 nm, and (iii) IR radiation having one or more wavelengths selected from the wavelength range of 750 - 950 nm; wherein the lighting system (1000) further comprises a control system (300), wherein the control system (300) is configured to control the first device radiation (111), the disinfection radiation (121), and the emission (407) of the first microbes (7), in dependence of one or more of a sensor (301), a timer, a user interface (310), and a predetermined program; wherein the sensor (301) is selected from the group comprising a movement sensor, a presence sensor, an activity detection sensor, a people counting sensor, a distance sensor, an ion sensor, a gas sensor, a volatile organic compound sensor, a pathogen sensor, an airflow sensor, a sound sensor, a temperature sensor, and a humidity sensor.

8. The lighting system (1000) according to claim 7, wherein the control system (300) is configured to control a microbe emission rate of the first microbes (7) in dependence of the disinfection radiation (121); wherein one or more of the following applies: (a) relative to a baseline emission rate, the microbe emission rate during at least part of the disinfection mode is higher than the baseline emission rate, and (b) relative to the baseline microbe emission rate, the microbe emission rate after the disinfection mode is higher than the baseline microbe emission rate.

9. The system (1000) according to any one of the preceding claims 6-8, wherein the control system (300) is configured to control the first device radiation (111), the disinfection radiation (121), and the microbe emission (407) of the first microbes (7) in dependence of information from a room context awareness system.

10. The lighting system (1000) according to any one of the preceding claims, wherein the microbe dispenser device (400) comprises a mist dispenser, wherein the mist dispenser is configured to dispense the first microbes (7); wherein the microbe dispenser device (400) comprises a cartridge holder (420), wherein the cartridge holder (420) is configured to detachably host a plurality of cartridges (425), wherein two or more of the plurality of cartridges (425) are filled with material differing in one or more of (a) types of first microbes (7) and (b) types of odor.

11. A lighting device (1200) comprising (i) the lighting system (1000) according to any one of the preceding claims and (ii) a housing (1250), wherein the housing (1250) encloses at least part of the light generating device (100) and at least part of the microbe dispenser device (400).

12. The lighting device (1200) according to claim 11, wherein the lighting device (1200) comprises the lighting system (1000) according to any one of the preceding claims 6-9, wherein the first beam (115) of first device radiation (111) has a first direction parallel to a first optical axis (O1) of the first beam (115); wherein the second beam (125) of disinfection radiation (121) has a second direction parallel to a second optical axis (O2) of the second beam (125), wherein the first direction and the second direction have a mutual angle selected from the range of 90-180°.

13. A method for indoor microbiome management, wherein the method comprises providing in an indoor space (3)
(a) a light generating device (100) providing in a microbic lighting mode a first beam (115) of first device radiation (111), and
(b) a microbe dispenser device (400) providing in a microbic application mode an emission (407) of first microbes (7) in a microbe emission region (415);
wherein the microbe dispenser device (400) has a microbe emission region (415) at least partly spatially overlapping with the first beam (115);
wherein a spectral power distribution of the first device radiation (111) is selected for promoting persistence of the first microbes (7) relative to second microbes, other than the first microbes (7).

14. The method according to claim 13, the method comprising: (b) the microbe dispenser device (400) providing in the microbic application mode a spray (410) of the first microbes (7) in the microbe emission region (415); wherein the microbic lighting mode (a) overlaps in time with the microbic application mode or (b) is subsequent in time to the microbic application mode.

15. The method according to any one of the preceding claims 13-14, further comprising: (a) directing in a disinfection mode at least part of disinfection radiation (121) to a ceiling (4); wherein the disinfection radiation (121) comprises UV radiation having one or more wavelengths selected from the wavelength range of 100 - 380 nm, visible near UV radiation having one or more wavelengths selected from the wavelength range of 380 - 495 nm, and IR radiation having one or more wavelengths selected from the wavelength range of 750 - 950 nm (b) directing in the microbic lighting mode at least part of the first beam (115) of first device radiation (111) to a floor (5), and (c) directing in the microbic application mode at least part of the emission (407) of the first microbes (7) to the floor (5).

## Patentansprüche

1. Beleuchtungssystem (1000) für eine Innenraumikrobiomverwaltung, wobei das Beleuchtungssystem (1000) eine Lichterzeugungsvorrichtung (100) und eine Mikrobenabgabevorrichtung (400) umfasst; wobei:
- die Mikrobenabgabevorrichtung (400) konfiguriert ist, um in einem mikrobiellen Anwendungsmodus eine Emission (407) von ersten Mikroben (7) bereitzustellen, wobei die Mikrobenabgabevorrichtung (400) einen Mikrobenemissionsbereich (415) aufweist;
- die Lichterzeugungsvorrichtung (100) konfiguriert ist, um in einem mikrobiellen Beleuchtungsmodus einen ersten Strahl (115) einer ersten Vorrichtungsstrahlung (111) bereitzustellen;
- eine spektrale Leistungsverteilung der ersten Vorrichtungsstrahlung (111) zum Fördern einer Persistenz der ersten Mikroben (7) relativ zu zweiten Mikroben mit Ausnahme der ersten Mikroben ausgewählt wird; und
- der Mikrobenemissionsbereich (415) und der erste Strahl (115) sich mindestens teilweise räumlich überschneiden.

2. Beleuchtungssystem (1000) nach Anspruch 1, wobei der mikrobielle Beleuchtungsmodus sich mit dem mikrobiellen Anwendungsmodus zeitlich überschneidet oder auf den mikrobiellen Anwendungsmodus zeitlich folgt.

3. Beleuchtungssystem (1000) nach einem der vorstehenden Ansprüche, wobei die spektrale Leistungsverteilung der ersten Vorrichtungsstrahlung (111) für eines oder mehrere von (i) Fördern eines Wachstums der ersten Mikroben (7), (ii) Deaktivieren von zweiten Mikroben mit Ausnahme der ersten Mikroben (7), (iii) stärkerem Deaktivieren von zweiten Mikroben als der ersten Mikroben (7) und (iv) Deaktivieren von Viren ausgewählt wird.

4. Beleuchtungssystem (1000) nach einem der vorstehenden Ansprüche, wobei die Mikrobenabgabevorrichtung (400) konfiguriert ist, um in dem mikrobiellen Anwendungsmodus einen Sprühnebel (410) der ersten Mikroben (7) bereitzustellen.

5. Beleuchtungssystem (1000) nach einem der vorstehenden Ansprüche, wobei (i) die Lichterzeugungsvorrichtung (100) konfiguriert ist, um in einem Standardbeleuchtungsmodus weiße erste Vorrichtungsstrahlung (111) bereitzustellen, und wobei (ii) die Lichterzeugungsvorrichtung (100) konfiguriert ist, um in dem mikrobiellen Beleuchtungsmodus weiße erste Vorrichtungsstrahlung (111) bereitzustellen; wobei eine relative spektrale Leistungsverteilung eines ersten Wellenlängenbereichs relativ zu der spektralen Leistungsverteilung in dem Wellenlängenbereich von 200-780 nm während mindestens eines Teils des mikrobiellen Beleuchtungsmodus höher als während mindestens eines Teils des Standardbeleuchtungsmodus ist, wobei:
- der erste Wellenlängenbereich den Bereich von 222 nm +/- 5 nm umfasst; oder
- der erste Wellenlängenbereich den Bereich von 270 nm +/- 10 nm umfasst; oder
- der erste Wellenlängenbereich den Bereich von 405 nm +/- 5 nm umfasst.

6. Beleuchtungssystem (1000) nach einem der vorstehenden Ansprüche, wobei die ersten Mikroben (7) aus der Gruppe ausgewählt sind, umfassend *Akkermansia, Bifidobacterium, Lachnospira, Alistipes, Bacteroides, Coprococcus, Viellonella, Faecalibacterium, Roseburia, Dialister, Sutterella, Methanobrevibacter, Lactobacillus, Parabacteroides, Prevotella, Agathobacter, Eubacterium, Ruminococcus, Nitrosomonas, Nitrosospira, Nitrosopumilus, Cenarchaeum, Nitrosoarchaeum, Nitrosocaldus, Caldiarchaeum, Acinetobacter, Alcaligenes, Arthrobacter, Azospirillum, Azotobacter, Bacillus, Beijerinckia, Enterobacter, Erwinia, Flavobacterium, Rhizobium, Serratia* und *Deinococcus.*

7. Beleuchtungssystem (1000) nach einem der vorstehenden Ansprüche, wobei die Lichterzeugungsvorrichtung (100) konfiguriert ist, um in einem Desinfektionsmodus Desinfektionsstrahlung (121) bereitzustellen, wobei die Desinfektionsstrahlung (121) eines oder mehrere von (i) UV-Strahlung, die eine oder mehrere Wellenlängen aufweist, die aus dem Wellenlängenbereich von 100-380 nm ausgewählt werden, (ii) sichtbarer Nah-UV-Strahlung, die eine oder mehrere Wellenlängen aufweist, die aus dem Wellenlängenbereich von 380-495 nm ausgewählt werden, und (iii) IR-Strahlung, die eine oder mehrere Wellenlängen aufweist, die aus dem Wellenlängenbereich von 750-950 nm ausgewählt werden, umfasst; wobei das Beleuchtungssystem (1000) ferner ein Steuerungssystem (300) umfasst, wobei das Steuerungssystem (300) konfiguriert ist, um die erste Vorrichtungsstrahlung (111), die Desinfektionsstrahlung (121) und die Emission (407) der ersten Mikroben (7) in Abhängigkeit von einem oder mehreren von einem Sensor (301), einem Zeitgeber, einer Benutzerschnittstelle (310) und einem zuvor bestimmten Programm zu steuern; wobei der Sensor (301) aus der Gruppe ausgewählt ist, umfassend einen Bewegungssensor, einen Anwesenheitssensor, einen Aktivitätserfassungssensor, einen Personenzählsensor, einen Entfernungssensor, einen Ionensensor, einen Gassensor, einen Sensor für flüchtige organische Verbindungen, einen Krankheitserregersensor, einen Luftstromsensor, einen Schallsensor, einen Temperatursensor und einen Feuchtigkeitssensor.

8. Beleuchtungssystem (1000) nach Anspruch 7, wobei das Steuerungssystem (300) konfiguriert ist, um eine Mikrobenemissionsrate der ersten Mikroben (7) in Abhängigkeit von der Desinfektionsstrahlung (121) zu steuern; wobei eines oder mehrere der Folgenden zutreffen: (a) relativ zu einer Basisemissionsrate ist die Mikrobenemissionsrate während mindestens eines Teils des Desinfektionsmodus höher als die Basisemissionsrate, und (b) relativ zu der Basisemissionsrate ist die Mikrobenemissionsrate nach dem Desinfektionsmodus höher als die Basisemissionsrate.

9. System (1000) nach einem der vorstehenden Ansprüche 6 bis 8, wobei das Steuerungssystem (300) konfiguriert ist, um die erste Vorrichtungsstrahlung (111), die Desinfektionsstrahlung (121) und die Mikrobenemission (407) der ersten Mikroben (7) in Abhängigkeit von Informationen aus einem Raumkontexterkennungssystem zu steuern.

10. Beleuchtungssystem (1000) nach einem der vorstehenden Ansprüche, wobei die Mikrobenabgabevorrichtung (400) eine Nebelabgabeeinrichtung umfasst, wobei die Nebelabgabeeinrichtung konfiguriert ist, um die ersten Mikroben (7) abzugeben; wobei die Mikrobenabgabevorrichtung (400) einen Kartuschenhalter (420) umfasst, wobei der Kartuschenhalter (420) konfiguriert ist, um eine Vielzahl von Kartuschen (425) abnehmbar aufzunehmen, wobei zwei oder mehr der Vielzahl von Kartuschen (425) mit Material gefüllt sind, das sich in einem oder mehreren von (a) Arten von ersten Mikroben (7) und (b) Geruchsarten unterscheidet.

11. Beleuchtungsvorrichtung (1200), umfassend (i) das Beleuchtungssystem (1000) nach einem der vorstehenden Ansprüche und (ii) ein Gehäuse (1250), wobei das Gehäuse (1250) mindestens einen Teil der Lichterzeugungsvorrichtung (100) und mindestens einen Teil der Mikrobenabgabevorrichtung (400) umschließt.

12. Beleuchtungsvorrichtung (1200) nach Anspruch 11, wobei die Beleuchtungsvorrichtung (1200) das Beleuchtungssystem (1000) nach einem der vorstehenden Ansprüche 6 bis 9 umfasst, wobei der erste Strahl (115) der ersten Vorrichtungsstrahlung (111) eine erste Richtung parallel zu einer ersten optischen Achse (O1) des ersten Strahls (115) aufweist; wobei der zweite Strahl (125) der Desinfektionsstrahlung (121) eine zweite Richtung parallel zu einer zweiten optischen Achse (O2) des zweiten Strahls (125) aufweist, wobei die erste Richtung und die zweite Richtung einen gegenseitigen Winkel aufweisen, der aus dem Bereich von 90-180° ausgewählt ist.

13. Verfahren für die Innenraumikrobiomverwaltung, wobei das Verfahren das Bereitstellen in einem Innenraum (3) umfasst
(a) einer Lichterzeugungsvorrichtung (100), die in einem mikrobiellen Beleuchtungsmodus einen ersten Strahl (115) der ersten Vorrichtungsstrahlung (111) bereitstellt, und
(b) einer Mikrobenabgabevorrichtung (400), die in einem mikrobiellen Anwendungsmodus eine Emission (407) von ersten Mikroben (7) in einem Mikrobenemissionsbereich (415) bereitstellt;
wobei die Mikrobenabgabevorrichtung (400) einen Mikrobenemissionsbereich (415) aufweist, der sich mindestens teilweise mit dem ersten Strahl (115) räumlich überschneidet;
wobei eine spektrale Leistungsverteilung der ersten Vorrichtungsstrahlung (111) zum Fördern der Persistenz der ersten Mikroben (7) relativ zu zweiten Mikroben mit Ausnahme der ersten Mikroben (7) ausgewählt wird.

14. Verfahren nach Anspruch 13, das Verfahren ferner umfassend: (b) die Mikrobenabgabevorrichtung (400) stellt in dem mikrobiellen Anwendungsmodus einen Sprühnebel (410) der ersten Mikroben (7) in dem Mikrobenemissionsbereich (415) bereit; wobei der mikrobielle Beleuchtungsmodus (a) sich mit dem mikrobiellen Anwendungsmodus zeitlich überschneidet oder (b) auf den mikrobiellen Anwendungsmodus zeitlich folgt.

15. Verfahren nach einem der vorstehenden Ansprüche 13 bis 14, ferner umfassend: (a) Richten von mindestens einem Teil der Desinfektionsstrahlung (121) in einem Desinfektionsmodus auf eine Decke (4); wobei die Desinfektionsstrahlung (121) UV-Strahlung, die eine oder mehrere Wellenlängen aufweist, die aus dem Wellenlängenbereich von 100-380 nm ausgewählt werden, sichtbare Nah-UV-Strahlung, die eine oder mehrere Wellenlängen aufweist, die aus dem Wellenlängenbereich von 380-495 nm ausgewählt werden, und IR-Strahlung, die eine oder mehrere Wellenlängen aufweist, die aus dem Wellenlängenbereich von 750-950 nm ausgewählt werden, umfasst; (b) Richten von mindestens einem Teil des ersten Strahls (115) der ersten Vorrichtungsstrahlung (111) in dem mikrobiellen Beleuchtungsmodus auf einen Boden (5), und (c) Richten von mindestens einem Teil der Emission (407) der ersten Mikroben (7) auf den Boden (5) in dem mikrobiellen Anwendungsmodus.

## Revendications

1. Système d'éclairage (1000) destiné à une gestion intérieure de microbiome, dans lequel le système d'éclairage (1000) comprend un dispositif générateur de lumière (100) et un dispositif distributeur de microbes (400) ; dans lequel :
- le dispositif distributeur de microbes (400) est configuré pour fournir dans un mode d'application microbienne une émission (407) de premiers microbes (7), dans lequel le dispositif distributeur de microbes (400) a une région d'émission de microbes (415) ;
- le dispositif générateur de lumière (100) est configuré pour fournir dans un mode d'éclairage microbien un premier faisceau (115) de premier rayonnement de dispositif (111) ;
- une distribution de puissance spectrale du premier rayonnement de dispositif (111) est sélectionnée pour promouvoir la persistance des premiers microbes (7) par rapport à des seconds microbes, autres que les premiers microbes ; et
- la région d'émission de microbes (415) et le premier faisceau (115) se chevauchent au moins partiellement dans l'espace.

2. Système d'éclairage (1000) selon la revendication 1, dans lequel le mode d'éclairage microbien chevauche dans le temps le mode d'application microbienne ou est postérieur dans le temps au mode d'application microbienne.

3. Système d'éclairage (1000) selon l'une quelconque des revendications précédentes, dans lequel la distribution de puissance spectrale du premier rayonnement de dispositif (111) est sélectionnée pour une ou plusieurs parmi (i) la promotion de croissance des premiers microbes (7), (ii) la désactivation de seconds microbes, autres que les premiers microbes (7), (iii) la désactivation plus forte des seconds microbes par rapport aux premiers microbes (7), et (iv) la désactivation de virus.

4. Système d'éclairage (1000) selon l'une quelconque des revendications précédentes, dans lequel le dispositif distributeur de microbes (400) est configuré pour fournir dans le mode d'application microbienne une pulvérisation (410) des premiers microbes (7).

5. Système d'éclairage (1000) selon l'une quelconque des revendications précédentes, dans lequel (i) le dispositif générateur de lumière (100) est configuré pour fournir dans un mode d'éclairage standard un premier rayonnement de dispositif (111) blanc, et dans lequel (ii) le dispositif générateur de lumière (100) est configuré pour fournir dans le mode d'éclairage microbien un premier rayonnement de dispositif (111) blanc ; dans lequel une distribution de puissance spectrale relative d'une première plage de longueur d'onde par rapport à la distribution de puissance spectrale dans la plage de longueur d'onde de 200 à 780 nm est pendant au moins une partie du mode d'éclairage microbien plus élevée que pendant au moins une partie du mode d'éclairage standard, dans lequel :
- la première plage de longueur d'onde comprend la plage de 222 nm +/- 5 nm ; ou
- la première plage de longueur d'onde comprend la plage de 270 nm +/- 10 nm ; ou
- la première plage de longueur d'onde comprend la plage de 405 nm +/- 5 nm.

6. Système d'éclairage (1000) selon l'une quelconque des revendications précédentes, dans lequel les premiers microbes (7) sont sélectionnés dans le groupe comprenant *Akkermansia, Bifidobacterium, Lachnospira, Alistipes, Bacteroides, Coprococcus, Viellonella, Faecalibacterium, Roseburia, Dialister, Sutterella, Methanobrevibacter, Lactobacillus, Parabacteroides, Prevotella, Agathobacter, Eubacterium, Ruminococcus, Nitrosomonas, Nitrosospira, Nitrosopumilus, Cenarchaeum, Nitrosoarchaeum, Nitrosocaldus, Caldiarchaeum, Acinetobacter, Alcaligenes, Arthrobacter, Azospirillum, Azotobacter, Bacillus, Beijerinckia, Enterobacter, Erwinia, Flavobacterium, Rhizobium, Serratia* et *Deinococcus.*

7. Système d'éclairage (1000) selon l'une quelconque des revendications précédentes, dans lequel le dispositif générateur de lumière (100) est configuré pour fournir dans un mode de désinfection un rayonnement de désinfection (121), dans lequel le rayonnement de désinfection (121) comprend un ou plusieurs parmi (i) un rayonnement UV ayant une ou plusieurs longueurs d'onde sélectionnées parmi la plage de longueur d'onde de 100 à 380 nm, (ii) un rayonnement visible UV proche ayant une ou plusieurs longueurs d'onde sélectionnées parmi la plage de longueur d'onde de 380 à 495 nm, et (iii) un rayonnement IR ayant une ou plusieurs longueurs d'onde sélectionnées parmi la plage de longueur d'onde de 750 à 950 nm ; dans lequel le système d'éclairage (1000) comprend en outre un système de commande (300), dans lequel le système de commande (300) est configuré pour commander le premier rayonnement de dispositif (111), le rayonnement de désinfection (121), et l'émission (407) des premiers microbes (7), en dépendance d'un ou plusieurs parmi un capteur (301), un temporisateur, une interface utilisateur (310) et un programme prédéterminé ; dans lequel le capteur (301) est sélectionné dans le groupe comprenant un capteur de mouvement, un capteur de présence, un capteur de détection d'activité, un capteur de comptage de personnes, un capteur de distance, un capteur d'ions, un capteur de gaz, un capteur de composés organiques volatils, un capteur de pathogènes, un capteur de débit d'air, un capteur de son, un capteur de température et un capteur d'humidité.

8. Système d'éclairage (1000) selon la revendication 7, dans lequel le système de commande (300) est configuré pour commander un taux d'émission de microbes des premiers microbes (7) en dépendance du rayonnement de désinfection (121) ; dans lequel un ou plusieurs de ce qui suit s'applique : (a) par rapport à un taux d'émission de ligne de base, le taux d'émission de microbes pendant au moins une partie du mode de désinfection est plus élevé que le taux d'émission de ligne de base, et (b) par rapport au taux d'émission de microbes de ligne de base, le taux d'émission de microbes après le mode de désinfection est plus élevé que le taux d'émission de microbes de ligne de base.

9. Système (1000) selon l'une quelconque des revendications 6 à 8 précédentes, dans lequel le système de commande (300) est configuré pour commander le premier rayonnement de dispositif (111), le rayonnement de désinfection (121), et l'émission de microbes (407) des premiers microbes (7) en dépendance d'informations provenant d'un système de reconnaissance de contexte de pièce.

10. Système d'éclairage (1000) selon l'une quelconque des revendications précédentes, dans lequel le dispositif distributeur de microbes (400) comprend un brumisateur, dans lequel le brumisateur est configuré pour distribuer les premiers microbes (7) ; dans lequel le dispositif distributeur de microbes (400) comprend un support de cartouche (420), dans lequel le support de cartouche (420) est configuré pour héberger de manière détachable une pluralité de cartouches (425), dans lequel deux ou plus parmi la pluralité de cartouches (425) sont remplies avec un matériau qui diffère dans un ou plusieurs parmi (a) les types de premiers microbes (7) et (b) les types d'odeur.

11. Dispositif d'éclairage (1200) comprenant (i) le système d'éclairage (1000) selon l'une quelconque des revendications précédentes et (ii) un logement (1250), dans lequel le logement (1250) renferme au moins une partie du dispositif générateur de lumière (100) et au moins une partie du dispositif distributeur de microbes (400).

12. Dispositif d'éclairage (1200) selon la revendication 11, dans lequel le dispositif d'éclairage (1200) comprend le système d'éclairage (1000) selon l'une quelconque des revendications 6 à 9 précédentes, dans lequel le premier faisceau (115) de premier rayonnement de dispositif (111) a une première direction parallèle à un premier axe optique (01) du premier faisceau (115) ; dans lequel le second faisceau (125) de rayonnement de désinfection (121) a une seconde direction parallèle à un second axe optique (O2) du second faisceau (125), dans lequel la première direction et la seconde direction ont un angle mutuel sélectionné dans la plage de 90 à 180°.

13. Procédé destiné à une gestion intérieure de microbiome, dans lequel le procédé comprend la fourniture dans un espace intérieur (3)
(a) d'un dispositif générateur de lumière (100) fournissant dans un mode d'éclairage microbien un premier faisceau (115) de premier rayonnement de dispositif (111), et
(b) d'un dispositif distributeur de microbes (400) fournissant dans un mode d'application microbienne une émission (407) de premiers microbes (7) dans une région d'émission de microbes (415) ;
dans lequel le dispositif distributeur de microbes (400) a une région d'émission de microbes (415) chevauchant au moins partiellement dans l'espace le premier faisceau (115) ;
dans lequel une distribution de puissance spectrale du premier rayonnement de dispositif (111) est sélectionnée pour promouvoir une persistance des premiers microbes (7) par rapport à des seconds microbes, autres que les premiers microbes (7).

14. Procédé selon la revendication 13, le procédé comprenant : (b) le dispositif distributeur de microbes (400) fournissant dans le mode d'application microbienne une pulvérisation (410) des premiers microbes (7) dans la région d'émission de microbes (415) ; dans lequel le mode d'éclairage microbien (a) chevauche dans le temps le mode d'application microbienne ou (b) est postérieur dans le temps au mode d'application microbienne.

15. Procédé selon l'une quelconque des revendications 13 à 14 précédentes, comprenant en outre : (a) le fait de diriger dans un mode de désinfection au moins une partie d'un rayonnement de désinfection (121) vers un plafond (4) ; dans lequel le rayonnement de désinfection (121) comprend un rayonnement UV ayant une ou plusieurs longueurs d'onde sélectionnées parmi la plage de longueur d'onde de 100 à 380 nm, un rayonnement visible UV proche ayant une ou plusieurs longueurs d'onde sélectionnées parmi la plage de longueur d'onde de 380 à 495 nm, et un rayonnement IR ayant une ou plusieurs longueurs d'onde sélectionnées parmi la plage de longueur d'onde de 750 à 950 nm (b) le fait de diriger dans le mode d'éclairage microbien au moins une partie du premier faisceau (115) de premier rayonnement de dispositif (111) vers un sol (5), et (c) le fait de diriger dans le mode d'application microbienne au moins une partie de l'émission (407) des premiers microbes (7) vers le sol (5).
